# NEW EUROPEAN PATENT SPECIFICATION

(11) **EP 1 556 009 B2**
(45) Date of publication and mention of the opposition decision: **21.07.2021**
(45) Mention of the grant of the patent: 24.12.2014
(21) Application number: 03772600.7
(22) Date of filing: 24.10.2003
(51) Int. Cl.: A61K 9/12

(54) **COSMETIC AND PHARMACEUTICAL FOAM**
KOSMETISCHER UND PHARMAZEUTISCHER SCHAUM
MOUSSE COSMETIQUE ET PHARMACEUTIQUE

(30) Priority: 25.10.2002 IL 15248602; 29.11.2002 US 429546 P
(43) Date of publication of application: 27.07.2005
(73) Proprietor: Foamix Pharmaceuticals Ltd., Rehovot 7670402 (IL)
(72) Inventor: TAMARKIN, Dov, Maccabim (IL); FRIEDMAN, Doron, Karmei-Yosef (IL); EINI, Meir, Ness Ziona (IL)
(74) Representative: Nack, Ralph
(86) International application number: PCT/IB2003/005527
(87) International publication number: WO 2004/037225

(56) References cited:
- EP-A2- 0 213 827
- WO-A-91/11991
- WO-A-03/051294
- WO-A1-98/31339
- WO-A1-03/005985
- US-A- 4 661 340
- US-A- 5 034 220
- US-A- 5 326 557
- US-A- 5 326 996
- US-A- 5 378 451
- US-A- 5 435 996
- US-A- 5 536 743
- US-A- 5 641 480
- US-A- 5 679 324
- US-A- 5 922 331
- US-A- 6 093 408
- US-A1- 2002 035 046
- US-A1- 2002 072 544
- US-A1- 2002 098 215
- US-B2- 6 423 323
- Ziolkowski, B.: "Moderne Aerosloschäume in der Kosmetik", Seifen-Öle-Fette-Wachse, vol. 112, no. 13, 1986, pages 427-429,
- Technical information for 'Cremophor A'
- Technical information for 'Luvitol EHO'
- Safety data sheet for Mineral Oil
- Information regarding 'Tegiloxan 350'
- Technical information for 'Luviquat Polymer Grades'
- Internet excerpt: Triethanolamine
- 'Diethyltoluamid' from 'Wikipedia, der freien Enzyklopädie'
- 'Dimethylphthalat' from 'Wikipedia, der freien Enzyklopädie'
- Screenshot der Internet-Site: http://www.leuna-tenside.de/2006_7_14_3143 /2006_8_7_5750/2006_8_7_241/cas-68439-49-6
- "Allantoin", Römpp, 23 September 2015 (2015-09-23), Retrieved from the Internet: URL:https://roempp.thieme.de/roempp4.0/do/ data/RD-01-01552
- Statement on use of Luviquat FC 370 as a water gelling agent

## Description

### FIELD OF THE INVENTION

The invention relates to an alcohol-free, cosmetic or pharmaceutical foam carrier and its use. More specifically, the invention relates to a cosmetic or pharmaceutical foam carrier suitable for inclusion of both water soluble and oil soluble pharmaceutical and cosmetic agents.

### BACKGROUND OF THE INVENTION

External topical administration is an important route for the administration of drugs in disease treatment. In external topical administration, the drug is absorbed into and/or through skin, mucous membrane or wound tissue. Many groups of drugs, including, for example, antibiotic, anti-fungal, anti-inflammatory, anesthetic, analgesic, anti-allergic, corticosteroid, retinoid and anti-proliferative medications are preferably administered in hydrophobic media, e.g. ointments or oils. However, due to the undesirable consistency of these hydrophobic carriers, their use is limited. For instance, ointments containing white petrolatum, e.g., Vaseline petroleum jelly, as the carrier often form an impermeable barrier, so that metabolic products and excreta from the wounds to which they are applied are not easily removed or drained away. Furthermore, it is difficult for the active drug dissolved in the carrier to pass through the white petrolatum barrier layer into the wound tissue, so the efficacy of the drug is reduced.

In addition, ointments and creams often do not create an environment for promoting respiration of the wound tissue and it is not favorable to the normal respiration of the skin. An additional disadvantage of petroleum jelly-based products relates to the greasy feeling left following their topical application onto the skin, mucosal membranes and wounds. Besides petroleum jelly, hydrophobic pharmaceutical carriers now in use include liquid paraffin, lanolin, beeswax, vegetable oil, glycerin monostearate, higher alcohols, polyethylene glycol and some emulsifying agents, which also have undesirable flow properties and skin feel.

Several hydrophobic liquid and semi-solid oils, e.g., mono- and polyunsaturated oils from vegetable and marine sources, mineral oils, silicone oils, and liquid hydrophobic plant-derived oils, are known for their therapeutic benefits when applied topically, yet, their application in liquid form is not practical. Oils can also contain essential nutritional constituents, such as oil-soluble vitamins (e.g., vitamin A and vitamin E), minerals and other therapeutically beneficial constituents. Another class of therapeutic oils includes mineral and silicon oils useful for the treatment of skin dehydration and other medical disorders, which oils are liquid at ambient temperature. Such therapeutic oils unfortunately, cannot be applied by users in amounts sufficient to exert therapeutic affects because of they typically are liquid at use temperatures.

Other pharmaceutical active ingredients are water-soluble and require a water component in the carrier.

While semi-solid cosmetic and pharmaceutical formulations, such as creams, lotions, gels and ointments are commonly used by consumers, new forms are desirable, in order to achieve better control of the application, while maintaining or bestowing the skin beneficial properties of such products. Thus, the development of a new composition, having breakable foam consistency when extruded out of a container and liquid properties when applied onto the skin is advantageous. Ideally a foam should contain hydrophobic substances (solvents), which can act as emollients and provide the skin with soothing and nourishing properties. However, such hydrophobic solvents are difficult to formulate into a lather-producing or foam-producing product because the hydrophobic solvents interfere with the lather forming ability of the surfactant. Furthermore, addition of oils and other emollients to topical formulations can result in an unpleasant or annoying skin residue.

Use of emulsions in foam compositions is known. Emulsion systems provide a two-phase system including lipophilic or hydrophobic components in one phase and hydrophilic components in the second phase. The foamed emulsion typically is an oil-in-water emulsion in which the hydrophobic component is dispersed in the aqueous continuous phase. Surfactants for reducing surface tension and emulsifiers for improving foam stability are included in the foam composition.

Foams and, in particular, foam emulsions are complicated systems which do not form under all circumstances. Slight shifts in foam emulsion composition, such as by the addition of active ingredients, may destabilize the foam. Furthermore, many emulsions do not provide the high foam capacity, foam stability and/or fast-breaking action under stress or temperatures that are desired in a topical foam composition.

A particularly desirable type of oil-containing foam is such wherein all or part of the oil phase comprises silicone oil. Silicone oil is known for its skin protective features and its incorporation in topical products is beneficial. However, it is not obvious to produce silicone oil-based foams, since many silicone oils possess anti-foaming properties.

US Pat. No. 6,126,920 discloses treatment of various skin diseases, and in particular, scalp psoriasis, using a foamable pharmaceutical composition containing a corticosteroid active substance, an aliphatic alcohol, water, a fatty alcohol, a surface-active agent, a propellant and a buffering agent. The foamable composition contains 40-90% w/w composition of an aliphatic alcohol. US Pat. No. 6,126,920 is typical of many compositions that use aliphatic alcohols in the foam composition. The alcohol promotes fast drying and thereby attempts to address the sticky feeling left by many topical formulations after application; however, alcohols, and in particular the methyl, ethyl and isopropyl alcohols preferred in the '920 patent, are defatting agents and may cause skin to become dry and cracked. Hence, the presence of aliphatic alcohol in a therapeutic foam for external topical administration as taught in US Pat. No. 6,126,920 is undesirable.

US Pat. No. 5,536,743 to Borgmandescribes a buffered non-flowing composition suitable for the treatment of bacterial vaginosis which contains metronidazole. Suitable formulations include oil-in-water emulsions including an internal oil phase of about 10-40 wt% oil and anionic, cationic or nonionic surfactants. Suitable components of the oleaginous phase include long chain alcohols, esters, and acids, vegetable and animal oils and waxes. No other stabilizing agents are disclosed for use in foam aerosol compositions.

EP 0,598,412 describes a composition that is useful for skin protection against drying and harsh environmental substances. The protection is derived from the inclusion of poly(tetrafluoroethylene) (PTFE) in the composition. The composition includes low levels of both hydrophilic emollients and hydrophobic emollients. The compositions include high levels of surfactants, including ionic surfactants, and co-emulsifiers resulting in thick emulsions which are not flowable, and thus providing products which are inefficient foamers (or non-foaming) and too thick for spreading over large skin areas.

US Pat. 6,423,323 describes an aqueous foam emulsion. The composition includes a hydrophobic phase including fatty acids, emulsifiers and co-emulsifiers, and an aqueous phase containing hydrophilic moisturizers and emulsifiers. An optional ingredient according to US Pat. 6,423,323 is one or more refatting substances, in preferable concentrations of 0.5 to 2%, if the product is to be used for normal skin; and 3 to 6% for dry skin. Addition of high levels of co-emulsifiers such as fatty alcohols and fatty acids suggest that the foam is not stable. No other stabilizing agents are disclosed.

US Pat. 5,635,469 describes a foamable cleansing liquid composition comprising about 0.05% to about 10% of an emollient, in addition to cleansing surfactants, humectants and water soluble cationic or nonionic polymers, but no propellants. Low density foams are achieved using a novel non-aerosol foam dispenser. The foaming is achieved by operating a manual pump, which is not convenient for operation. Emollients and humectants are included to improve the level of hydration and/or lipid content of the skin. However, the patent notes that emollients and humectants interfere with the lather forming ability of the surfactant.

US Pat. 6,113,888 teaches a single water phase composition comprising a self-tanning agent, a nitrogen-free polymer, a nitrogen-free surfactant, and water.

US Pat. 5,679,324 to Lisboa pertains to an aerosol foamable fragrance composition, translucent in its pre-dispensed state, which forms a fast breaking foam. Apparently the foam breaks spontaneously upon discharging from an aerosol container (with no need of any rubbing or sheer force application), thus, making is impractical for spreading over a skin surface. The composition contains surfactant, a propellant, a fragrance, a thickener, and a cosmetic vehicle (preferably water) wherein the ratio of the surfactant to propellant is from about 1:1 to about 1:10. Emollients including silicone oils, mineral oils and hydrocarbon oils may be included.

US Pat. 6,251,369 discloses foamable dental fluoride compositions containing a water-soluble fluoride component, whereby said compositions include an oil in water emulsion. However, the patent fails to specify the identity or concentration of the oil component of the emulsion; and none of the compositions presented in the examples contain any oil component.

US Pat. 5,961,957 describes a barrier foam composition comprising from 70 to 90% of water, from 7 to 9% of butane, from 2 to 4% of glyceryl monostearate, from 1.5 to 3.50% of dimethicone copolyol (a water-soluble silicone compound), from 1 to 3% of propane, from 0.5 to 2.5% of lanolin, from 0.5 to 2.5% of stearic acid and from 0.05 to 1.05% of at least one of methylchloroisothiazolinone and methylisothiazolinone.

A few dermatological foam products are available on the market.

Olux^{™} Foam, produced by Connetics, Inc., contains clobetasol propionate. Each gram of Olux^{™} Foam contains 0.5 mg clobetasol propionate, USP, in a thermolabile foam, which consists of ethanol (60%), purified water, propylene glycol, cetyl alcohol, stearyl alcohol, polysorbate 60, citric acid, and potassium citrate. It is dispensed from an aluminum can pressurized with a hydrocarbon propellant (propane/butane). Luxiq^{™} is another corticostroid foam medication, containng 1.2 mg betamethasone valerate per gram, in a vehicle, comprising ethanol (60.4%), purified water, propylene glycol, cetyl alcohol, stearyl alcohol, polysorbate 60, citric acid, and potassium citrate, and pressurized with a hydrocarbon propellant.

Cortifoam, a hydrocortisone acetate rectal foam is produced by Schwartz Pharma GmbH, wherein the hydrocortisone is present at 10% In a foam vehicle.

Nonmedicinal ingredients of Cortifoam Include cetyl alcohol, ethoxylated stearyl alcohol. methylparaben, polyoxyethylene-10 stearyl ether, propylene glycol, propylparaben, triethanolamine, water, and inert propellants, isobutene, and propane.

US 5,326,557 and US 5,435,996 disclose moisturizing compositions for application to human skin which contain oxyethylene functional organosilanes. When used in skin care applications, these compounds exhibit humectant properties. The oxyethylene functional organosilane is a compound having the formula RSiR'3 in which R is the radical -O(CH₂CH₂O)xR"; R' is an R group or an alkyl radical having one to six carbon atoms; R" is a radical such as hydrogen; an alkyl group of one to six carbon atoms; and an aryl group; and x is an integer having a value of six to thirty.

US 2002/072544 discloses fine emulsions comprising at least one W/O emulsifier and at least one hydrophilic component, and a process for their preparation, The fine emulsions are obtainable by converting a W/O preemulslon comprising at least one W/O emulsifier into an O/W fine emulsion by adding at least one hydrophilic component and, where appropriate, changing the temperature. Suitable W/O emulsifiers are preferably sorbitol esters. The fine emulsions may be spray emulsions for cosmetic and pharmaceutical applications.

US 6,093,408 discloses compositions and methods for treatment of human skin. Such methods Include those for regulation sebum on facial skin of a human subject, comprising applying to said subject an emulsion composition comprising (a) an anhydrous silicone mixture comprising (i) a ethylene oxide/propylene oxide silicone copolymer; (ii) an ethylene oxide silicone copolymer; (iii) a silicone gum; and (iv) a silicone fluid; and (b) water.

US 5,922,331 discloses a skin cream composition for protection against lumpiness, edema, and other effects of liposuction and cosmetic surgery, as well as increasing the smoothness of the skin. In general, the skin cream composition comprises: water, and emulsified and dispersed in the water: (1) a long-chain fatty acid ester of ascorbic acid; (2) a short-chain carboxylic acid ester of tocopherol; (3) a glyceryl ester complex comprising at least one glyceryl ester selected from the group consisting of glyceryl linoleate, glyceryl linolenate, and glyceryl arachidonate; (4) a first complex consisting essentially of water, propylene glycol, lecithin, caffeine benzoate, and palmitoyl carnitine; (5) a second complex consisting essentially of water, caffeine, carnitine, and hydrolysed glycosaminoglycans; (6) a third complex consisting essentially of glycerol, butcher broom extract, passion flower extract, glycogen, hydrolysed collagen, and PEG 6-32; (7) calendula extract; (8) a water-glycol extract of chamomile; (9) hydrophilic microcapsules; (10) lipophilic microcapsules; and (11) microcapsules comprising methylsilanol elastinate. Other, optional cosmetic ingredients and ancillary ingredients can also be used.

US 5,641,480 discloses hair care compositions containing heteroatom containing alkyl aldonamide compounds and hair conditioning agents. These heteroatom containing alkyl aldonamides provide enhanced stability and/or enhanced viscosity relative to the use of other known thickeners or non-heteroatom containing aldonamides.

US 2002/098215 discloses oil-in-water nanoemulsions comprising oil globules with an average size of less than 150 nm and comprising at least one oil, at least one amphiphilic lipid, and at leat one nonionic polymer comprising at least one hydrophobic block and at least one hydrophilic block. Processes comprising such oll-in-water nanoemulsions are also disclosed.

US 2002/035046 discloses personal care compositions suitable for use in skin care applications, which deliver and/or deposit various benefit agents into and onto the skin and which are relatively non-irritating and thus suitable for use by people having sensitive skin and eyes comprising at least one ester and a water dispersible component.

Thus, foam compositions for topical treatment, containing higher concentrations of oils, and which do not comprise alcohol are still desirable. Foam compositions that are robust and suitable for inclusion of a wide range of active ingredients are desired.

### SUMMARY OF THE INVENTION

Despite the commonly known fact that hydrophobic solvents are difficult to formulate into a lather-producing or foam-producing product and that addition of conventional hydrophobic solvents interferes with the lather forming ability of the surfactant, we have surprisingly discovered a series of foamable carrier compositions, which, upon admixing with a liquefied gas propellant in an aerosol container, produces a foamable composition that is suitable for topical administration. Upon discharge from an aerosol container, the composition forms a breakable foam, which is rich and creamy in appearance, and show very fine bubble structure. The foam does not break down immediately upon discharge, however, it collapses to spread easily onto a skin area upon slight rubbing.

In one or more embodiments of the present invention, the alcohol-free cosmetic or pharmaceutical foamable carrier composition includes water, a liquid, non-volatile hydrophobic solvent, a foam adjuvant agent selected from the group consisting of fatty acids and fatty alcohols, a surface-active agent and a water gelling agent. Such foamable carriers, when placed in an aerosol container and combined with a liquefied gas propellant, create an oil in water emulsion, which, upon release from the aerosol container, provides a therapeutically beneficial foam product. The foam retains its structure for a time sufficient for a user to apply and to rub the foam into the skin. The foam has a very low yield strength and, hence, it breaks upon touch and makes rubbing easy and efficient, and its application even.

In one or more embodiments of the present invention, the foamable carrier composition the hydrophobic solvent content is about 2-5% and has a composition as follows:
Class A Composition:
   - about 2-5% hydrophobic solvent;
   - about 80-98% water;
   - about 0.1% to 5% foam adjuvant agent;
   - about 0.1 % to 5% surface-active agent; and
   - about 0.1 % to 5% water gelling agent.

In one or more embodiments of the present invention, the foamable composition the hydrophobic solvent content is about 5-10% and has a composition as follows:
Class B Composition:
   - about 5-10% hydrophobic solvent;
   - about 75-95% water;
   - about 0.1 % to 5% foam adjuvant agent;
   - about 0.1 % to 5% surface-active agent; and
   - about 0.1 % to 5% water gelling agent.

In one or more embodiments of the present invention, the foamable composition the hydrophobic solvent content is about 10-20% and has a composition as follows:
Class C Composition:
   - about 10-20% hydrophobic solvent;
   - about 60-90% water;
   - about 0.1% to 5% foam adjuvant agent;
   - about 0.1% to 5% surface-active agent; and
   - about 0.1% to 5% water gelling agent.

In one or more embodiments of the present invention, the foamable composition the hydrophobic-solvent content is about 20-75% and has a composition is follows:
Class D Composition:
   - about 20-75% hydrophobic solvent;
   - about 25-75% water;
   - about 0.1% to 5% foam adjuvant agent;
   - about 0.1% to 5% surface-active agent; and
   - about 0.1 % to 5% water gelling agent.

All % values are provided on a weight (w/w) basis, based on the composition with out propellant (unless otherwise specified).

The cosmetic or pharmaceutical foamable carrier composition is liquid. The foamable of the present invention does not contain short chain aliphatic alcohols, making it non-irritating and non-drying. Alcohols penetrate the skin's protective barrier and break down the intercellular matrix. In a recent publication by the American Academy of Dermatology (AAD), titled "Facing the Facts about Skin Care Products" it is stated "[i]ndividuals with dry skin should avoid astringents and any product with alcohol because they easily strip away moisture from the skin" (see: www.aad.org/PressReleases FacingFacts.html). Another AAD publication, titled "Sensitive About Your Skin?", recommends to "[a]void solvents that penetrate the skin including, propylene glycol and ethanol" (see: www.aad.org/PressReleases/sensitive.html).

The alcohol-free foam carrier is formulated as an oil-in-water or water-in-oil emulsion, so that it is suitable for inclusion of either water-soluble and oil soluble active agents (or both). The foamable carrier composition of the present invention, when admixed with a propellant substance in an amount of about 5-25% by weight of the total composition in an aerosol container, produces lightweight breakable foam, suitable for facile application onto the skin, and other body areas, which may accept topically-applied products. Since the propellant, in the pressurized container is in liquid state, upon admixing the foamable carrier composition with the propellant, a stable emulsion, comprising the oil and the propellant (jointly as the "oil phase" component of such emulsion) is formed.

In one or more embodiments of the present invention, an alcohol-free cosmetic or pharmaceutical product is provided. The product includes a foam carrier composition according to one or more embodiments of the present invention and an active cosmetic or pharmaceutical ingredient in a therapeutically effective concentration. Cosmetic and pharmaceutical agents can be included in each of the compositions described above and in the detailed description that follows. Pharmaceutical products are intended for topical treatment of human and animal skin disorders, or any other disorder, that requires topical application of a drug. Cosmetic products are intended for beautifying the skin and improving its appearance.

Cosmetic and medical disorders that are best treated using the alcohol-free foam carrier and the alcohol-free cosmetic or pharmaceutical product are identified, and the advantages of such carrier and products is demonstrated as compared to currently available options.

The foam of the present invention is advantageous to current options, for one or more of the following reasons:
(1) The foam is lightweight and thus, economical.
(2) The foam contains a hydrophobic solvent, in any desirable concentration, which provides a refatting and skin soothing effect, as well as a carrier for hydrophobic active agents.
(3) The foam contains silicone oil in a therapeutically effective concentration
(4) The foam includes active agent, both water soluble and oil soluble.
(5) The foam is easily spreadable, allowing treatment of large areas such as the arms, back, legs and the breast.
(6) Due to its flow properties, it spreads effectively into folds and wrinkles, providing uniform distribution of the active agent without the need of extensive rubbing and absorbs into the skin.

### BRIEF DESCRIPTION OF THE DRAWINGS

A more complete appreciation of the present invention and many of its advantages will be understood by reference to the following detailed description when considered in connection with the following drawings, which are presented for the purpose of illustration only are not intended to limit the scope of the appended claims, and in which:
Figure 1 illustrates the improvement in the treatment of psoriasis using Bethasone valerate 0.12% foam; and
Figure 2 illustrates the improvement in the treatment of atopic dermatitis using Bethasone valerate 0.12% foam.

### DETAILED DESCRIPTION OF THE INVENTION

### Hydrophobic solvent

A hydrophobic solvent according to the present invention is a liquid material having solubility in distilled water at ambient temperature of less than about 1 gm per 100 mL, more preferable less than about 0.5 gm per 100 mL, and most preferably less than about 0.1 gm per 100 mL. It is liquid at ambient temperature.

The total content of hydrophobic solvent may vary from 2% to 75% (w/w) of the foamable composition. However, different ranges (herein "composition classes A-D") have been designated, in order to facilitate a choice of an appropriate class, according to the anticipated cosmetic or pharmaceutical need. As a rule of thumb, higher hydrophobic solvent concentrations are more appropriate for the treatment of dry skin, and/or for the treatment of a disease, which is more responsive to drugs delivered in an oily vehicle. Likewise, the higher oil-content composition classes provide an enhanced occlusive effect, which in turn induces the skin penetration of an active agent. Another consideration relates to user acceptance of a product containing a high concentration of the hydrophobic solvent (from about 25% of the composition), which would leave some oily feeling post-application. Thus, a particular composition of the present invention is selected having a hydrophobic solvent concentration in view of the target population and its specific needs.

In one or more embodiments of the present invention, the hydrophobic solvent is mineral oil. Mineral oil (Chemical Abstracts Service Registry number 8012-95-1) is a mixture of aliphatic, naphthalenic, and aromatic liquid hydrocarbons that are derived from petroleum. It is typically liquid; its viscosity is in the range of about 35 CST to about 100 CST (at 40°C), and its pour point (the lowest temperature at which an oil can be handled without excessive amounts of wax crystals forming) is below 0°C. By contrast, white petrolatum, also termed "Vaseline", is disadvantageous, due to its waxy nature. It is known to leave waxy and sticky feeling after application and occasionally stain cloths. Thus, white petrolatum and other semi-solid oils are not a preferred hydrophobic solvent according to the present invention.

Yet another preferred hydrophobic solvents are liquid oils from vegetable, marine or animal sources. By way of example, the unsaturated oil may be selected from the group consisting of olive, corn, soybean, canola, cottonseed, coconut, sesame, sunflower, borage seed, *syzigium aromaticum,* hempseed, herring, cod-liver, salmon, flaxseed, wheat germ and evening primrose oils and mixtures thereof, at any proportion.

A particularly preferred class of oils includes polyunsaturated oils, e.g., esters, and in particular glyceryl esters, of omega-3 and omega-6 fatty acids. Examples of such polyunsaturated fatty acids are linoleic and linolenic acid, gamma-linoleic acid (GLA), eicosapentaenoic acid (EPA) and docosahexaenoic acid (DHA). Thus, in one or more embodiments of the present invention the hydrophobic solvent includes at least 6% by weight foamable composition of an oil selected from omega-3 oil, omega-6 oil, and mixtures thereof.

Another class of oils suitable for use as a phydrophobic solvent is liquid hydrophobic plant-derived oils, or essential oils, e.g. "therapeutic oils" containing active biologically occurring molecules that have a therapeutic effect when applied topically. Examples of such oils include rosehip oil, which contain retinoids and is known to reduce acne and post-acne scars, and tea tree oil, which possess antibacterial, antifungal and antiviral properties. Other examples of essential oils are oils of basil, camphor, cardamom, carrot, citronella, clary sage, clove, cypress, frankincense, ginger, grapefruit, hyssop, jasmine, lavender, lemon, mandarin, marjoram, myrrh, neroli, nutmeg, petitgrain, sage, tangerine, vanilla, verbena, as well as any other therapeutically beneficial oil, know in the art of herbal medication.

In one or more embodiments of the present invention, the hydrophobic solvent is an "emollient". An emollient is a hydrophobic agent that softens, smoothens and improves lipid content of the skin or other mucous membranes. In one or more embodiments of the present invention, the emollient is a liquid. Without derogating the generality of this definition, examples of suitable emollients for use include isostearic acid derivatives, isopropyl palmitate, lanolin oil, diisopropyl dimerate, diisopropyl adipate, dimethyl isosorbide, maleated soybean oil, octyl palmitate, isopropyl isostearate, cetyl lactate, cetyl ricinoleate, tocopheryl acetate, acetylated lanolin alcohol, cetyl acetate, phenyl trimethicone, glyceryl oleate, tocopheryl linoleate, wheat germ glycerides, arachidyl propionate, myristyl lactate, decyl oleate, propylene glycol ricinoleate, isopropyl lanolate, pentaerythrityl tetrastearate, neopentylglycol dicaprylate/dicaprate, hydrogenated coco-glycerides, isononyl isononanoate, isotridecyl isononanoate, myristyl myristate, triisocetyl citrate, octyl dodecanol, octyl hydroxystearate and mixtures thereof. Other examples of other suitable emollients can also be found in the Cosmetic Bench Reference, pp. 1.19-1.22 (1996). In one or more embodiments, the hydrophobic solvent is a mixture of a mineral oil or silicone oil and an emollient.

In one or more embodiments of the present invention, silicone oil is a component of the hydrophobic solvent. Silicone oils are used in the foamable compositions due to their known skin protective and occlusive properties. Suitable silicone oils for use in the invention include non-volatile silicones, such as polyalkyl siloxanes, polyaryl siloxanes, polyalkylaryl siloxanes and polyether siloxane copolymers, polydimethylsiloxanes (dimethicones) and poly(dimethylsiloxane)-(diphenyl-siloxane) copolymers. These are preferably chosen from cyclic or linear polydimethylsiloxanes containing from about 3 to about 9, preferably from about 4 to about 5, silicon atoms. Volatile silicones such as cyclomethicones can also be used. Water-soluble silicones, such as dimethicone copolyol are not included in the definition of silicone oils (as hydrophobic solvents) according to the present invention.

In one or more embodiments of the present invention, the composition comprises at least 2% (w/w foamable composition) silicone oil, alone or as part of the hydrophobic solvent. Yet, in other embodiments, the composition comprises at least 5% (w/w) silicone oil alone or as part of the hydrophobic solvent.

The hydrophobic solvent of the present invention may comprise a mixture of two or more of the above hydrophobic solvents in any proportion.

### Foam adjuvant agents

Foam adjuvants are included in the foamable compositions of the present invention to increase the foaming capacity of surfactants and/or to stabilize the foam. In one or more embodiments of the present invention, the foam adjuvant agents includes fatty alcohols having 15 or more carbons in their carbon chain, such as cetyl alcohol and stearyl alcohol (or mixtures thereof). Other examples of fatty alcohols are arachidyl alcohol (C20), behenyl alcohol (C22), 1-triacontanol (C30), as well as alcohols with longer carbon chains (up to C50). Fatty alcohols, derived from beeswax, including a mixture of alcohols, a majority of which has at least 20 carbon atoms in their carbon chain, are especially well suited as foam adjuvant agents according to the present invention. The concentration of the fatty alcohol, required to support the foam system is inversely related to the length of its carbon chains.

In one or more embodiments of the present invention, the foam adjuvant agent includes fatty acids having 16 or more carbons in their carbon chain, such as hexadecanoic acid (C16) stearic acid (C18), arachidic acid (C20), behenic acid (C22), octacosanoic acid (C28), as well as fatty acids with longer carbon chains (up to C50), or mixtures thereof.

Optionally, the carbon atom chain of the fatty alcohol or the fatty acid may have at least one double bond. A further class of foam adjuvant agent according to the present invention comprises a long chain fatty alcohol or fatty acid, wherein the carbon atom chain is branched. The carbon chain of the fatty acid or fatty alcohol can be substituted with a hydroxyl group, such as 12-hydroxy stearic acid.

The foam adjuvant agent according to one or more embodiments of the present invention includes a mixture of fatty alcohols, fatty acids and hydroxy fatty acids and derivatives thereof in any proportion, providing that the total amount is 0.1 % to 5% (w/w) of the carrier mass. More preferably, the total amount is 0.4% - 2.5% (w/w) of the carrier mass.

While fatty alcohols and fatty acids serve to stabilize the resultant foam composition, they often provide additional therapeutic properties. Long chain saturated and mono unsaturated fatty alcohols, e.g., stearyl alcohol, erycyl alcohol, arachidyl alcohol and docosanol have been reported to possess antiviral, anti infective, anti-proliferative and anti-inflammatory properties (US Patent No. 4,874,794). Longer chain fatty alcohols, e.g., tetracosanol, hexacosanol, heptacosanol, octacosanol, triacontanol, etc. are also known for their metabolism modifying properties and tissue energizing properties. Long chain fatty acids have also been reported to possess anti-infective characteristics. Thus, the pharmaceutical or cosmetic carrier, containing the foam adjuvant agent of the present invention provides an extra therapeutic benefit in comparison with currently used vehicles, which are inert and non-active.

### Surface-active agents

Surface-active agents, according to the present invention include any agent linking oil and water in the composition.

The surface-active agent is suitably selected from anionic, cationic, nonionic, zwitterionic, amphoteric and ampholytic surfactants, as well as mixtures of these surfactants. Such surfactants are well known to those skilled in the pharmaceutical and cosmetic formulation art. Nonlimiting examples of possible surfactants include polysorbates, such as polyoxyethylene (20) sorbitan monostearate (Tween 60) and poly(oxyethylene) (20) sorbitan monooleate (Tween 80); poly(oxyethylene) (POE) fatty acid esters, such as Myrj 45, Myrj 49 and Myrj 59; poly(oxyethylene) alkylyl ethers, such as poly(oxyethylene) cetyl ether, poly(oxyethylene) palmityl ether, polyethylene oxide hexadecyl ether, polyethylene glycol cetyl ether, brij 38, brij 52, brij 56 and brij W1; sucrose esters, partial esters of sorbitol and its anhydrides, such as sorbitan monolaurate and sorbitan monolaurate; mono or diglycerides, isoceteth-20, sodium methyl cocoyl taurate, sodium methyl oleoyl taurate, sodium lauryl sulfate, triethanolamine lauryl sulfate and betaines.

A combination of surface active agents is possible. Any surface-active agent or combinations thereof may be used as surface-active agent. According to one or more embodiments of the present invention, the surface-active agent (or agents) has an HLB of higher than 9.

In one or more embodiments of the present invention, the surface-active agent is selected from the groups of non ionic surfactants, cationic surfactants, amphoteric and zwitterionic surfactants, and, in particular, the surface-active agent is a non-ionic surfactant. Ionic surfactants (including cationic, anionic, amphotheric and zwitterionic surfactants) are known to be skin irritants. Therefore, non-ionic surfactants are preferred in applications including sensitive skin such as found in most dermological disorders. We have surprisingly found that non-ionic surfactants alone provide foams of excellent quality, i.e. a score of "E" according to the grading scale discussed below.

In one or more embodiments of the present invention, the surface active agent is solely non-ionic, comprising one or more non-ionic surfactants.

In one or more embodiments of the present invention, the surface active agent include a ratio of non-ionic surfactants to ionic surfactants in the range of 100:1 to 6:1; in some embodiments the non-ionic to ionic surfactant ratio is greater than 6:1, or greater than 8:1; or greater than 14:1, or greater than 16:1, or greater than 20:1.

Exemplary non-ionic surfactants include polyethoxylated fatty acids, fatty acid diesters, polyethylene glycol glycerol fatty acid esters, alcohol-oil transesterification products, polyglycerized fatty acids, sterol and sterol derivatives, polyethylene glycol sorbitan fatty acid esters, polyethylene glycol alkyl ethers, sugar esters, polyethylene glycol alkyl phenols, polyoxyethylene-polyoxypropylene block copolymers, sorbitan fatty acid esters and lower alcohol fatty acid esters.

Although polyethylene glycol (PEG) itself does not function as a surfactant, a variety of PEG-fatty acid esters have useful surfactant properties. Exemplary monoesters include esters of lauric acid, oleic acid, and stearic acid, e.g., PEG-8 laurate, PEG-8 oleate, PEG-8 stearate, PEG-9 oleate, PEG-10 laurate, PEG-10 oleate, PEG-12 laurate, PEG-12 oleate, PEG-15 oleate, PEG-20 laurate and PEG-20 oleate. Polyethylene glycol fatty acid diesters suitable for use as non-ionic surfactants in the compositions of the present invention include PEG-20 dilaurate, PEG-20 dioleate, PEG-20 distearate, PEG-32 dilaurate and PEG-32 dioleate. Suitable polyethylene glycol glycerol fatty acid esters include PEG-20 glyceryl laurate, PEG-30 glyceryl laurate, PEG-40 glyceryl laurate, PEG-20 glyceryl oleate, and PEG-30 glyceryl oleate.

A large number of surfactants of different degrees of hydrophobicity or hydrophilicity can be prepared by reaction of alcohols or polyalcohols with a variety of natural and/or hydrogenated oils. Most commonly, the oils used are castor oil or hydrogenated castor oil, or an edible vegetable oil such as corn oil, olive oil, peanut oil, palm kernel oil, apricot kernel oil, or almond oil. Preferred alcohols include glycerol, propylene glycol, ethylene glycol, polyethylene glycol, sorbitol, and pentaerythritol. Among these alcohol-oil transesterified surfactants, preferred hydrophilic surfactants are PEG-35 castor oil (Incrocas-35), PEG-40 hydrogenated castor oil (Cremophor RH 40), PEG-25 trioleate (TAGAT® TO), PEG-60 corn glycerides (Crovol M70), PEG-60 almond oil (Crovol A70), PEG-40 palm kernel oil (Crovol PK70), PEG-50 castor oil (Emalex C-50), PEG-50 hydrogenated castor oil (Emalex HC-50), PEG-8 caprylic/capric glycerides (Labrasol), and PEG-6 caprylic/capric glycerides (Softigen 767). Preferred hydrophobic surfactants in this class include PEG-5 hydrogenated castor oil, PEG-7 hydrogenated castor oil, PEG-9 hydrogenated castor oil, PEG-6 corn oil (Labrafil® M 2125 CS), PEG-6 almond oil (Labrafil® M 1966 CS), PEG-6 apricot kernel oil (Labrafil® M 1944 CS), PEG-6 olive oil (Labrafil® M 1980 CS), PEG-6 peanut oil (Labrafil® M 1969 CS), PEG-6 hydrogenated palm kernel oil (Labrafil® M 2130 BS), PEG-6 palm kernel oil (Labrafil® M 2130 CS), PEG-6 triolein (Labrafil® b M 2735 CS), PEG-8 corn oil (Labrafil® WL 2609 BS), PEG-20 corn glycerides (Crovol M40), and PEG-20 almond glycerides (Crovol A40). The latter two surfactants are reported to have HLB values of 10, which is generally considered to be the approximate border line between hydrophilic and hydrophobic surfactants.

Alcohol-oil transesterification derivatives of oil soluable vitamins (e.g., vitamins A, D, E, K, etc.), such as tocopheryl PEG-100 succinate (TPGS, available from Eastman), are also suitable surfactants.

Polyglycerol esters of fatty acids are also suitable non-ionic surfactants for the present invention. Among the polyglyceryl fatty acid esters, exemplary use hydrophobic surfactants include polyglyceryl oleate (Plurol Oleique), polyglyceryl-2 dioleate (Nikkol DGDO), and polyglyceryl-10 trioleate. Preferred hydrophilic surfactants include polyglyceryl-10 laurate (Nikkol Decaglyn 1-L), polyglyceryl-10 oleate (Nikkol Decaglyn 1-O), and polyglyceryl-10 mono, dioleate (Caprol® PEG 860), Polyglyceryl polyricinoleates (Polymuls) are hydrophilic and hydrophobic surfactants of this class.

Sterols and derivatives of sterols are suitable surfactants for use in the present invention. These surfactants can be hydrophilic or hydrophobic. Preferred derivatives include the polyethylene glycol derivatives. An exemplary hydrophobic surfactant in this class is cholesterol. An exemplary hydrophilic surfactant in this class is PEG-24 cholesterol ether (Solulan C-24).

A variety of PEG-sorbitan fatty acid esters are suitable for use as non-ionic surfactants in the present invention. In general, these surfactants are hydrophilic, although several hydrophobic surfactants of this class can be used. Among the PEG-sorbitan fatty acid esters, exemplary hydrophilic surfactants include PEG-20 sorbitan monolaurate (Tween-20), PEG-20 sorbitan monopalmitate (Tween-40), PEG-20 sorbitan monostearate (Tween-60), and PEG-20 sorbitan monooleate (Tween-80).

Ethers of polyethylene glycol and alkyl alcohols are suitable non-ionic surfactants for use in the present invention. Exemplary hydrophobic ethers include PEG-3 oleyl ether (Volpo 3) and PEG-4 lauryl ether (Brij 30).

The polyoxyethylene-polyoxypropylene (POE-POP) block copolymers are a unique class of polymeric surfactants. The unique structure of the surfactants, with hydrophilic POE and hydrophobic POP moieties in well-defined ratios and positions, provides a wide variety of surfactants suitable for use in the present invention. These surfactants are available under various trade names, including Synperonic PE series (ICI), Pluronic® series (BASF), Emkalyx, Lutrol (BASF), Supronic, Monolan, Pluracare, and Plurodac. The generic term for these polymers is "poloxamer" (CAS 9003-11-6). Exemplary hydrophilic surfactants of this class include Poloxamers 108, 188, 217, 238, 288, 338, and 407. Exemplary hydrophobic surfactants in this class include Poloxamers 124, 182, 183, 212, 331, and 335.

Sorbitan esters of fatty acids are suitable non-ionic surfactants for use in the present invention. Among these esters, preferred hydrophobic surfactants include sorbitan monolaurate (Arlacel 20), sorbitan monopalmitate (Span-40), sorbitan monooleate (Span-80), sorbitan monostearate, and sorbitan tristearate.

Esters of lower alcohols (C₂ to C₄) and fatty acids (C₈ to C₁₈) are suitable non-ionic surfactants for use in the present invention. Among these esters, preferred hydrophobic surfactants include ethyl oleate (Crodamol EO), isopropyl myristate (Crodamol IPM), and isopropyl palmitate (Crodamol IPP).

In one or more embodiments of the present invention, the surface-active agent comprise mono-, di- and tri-esters of sucrose with food fatty acids (sucrose esters), prepared from sucrose and methyl and ethyl esters of food fatty acids or by extraction from sucroglycerides. Exemplary sucrose esters include sucrose monopalmitate and sucrose monolaurate. Suitable sucrose esters include those having a high monoester content, which have higher HLB values.

In one or more embodiments of the present invention, a combination of a non-ionic surfactant and an anionic surfactant (such as sodium lauryl sulphate) is employed, at a ratio of between 1:1 and 20:1, or at a ratio of 4:1 to 10:1. The resultant foam has a low specific gravity, e.g., less than 0.1g/ml, which upon rubbing (shear stress) onto the skin collapses easily, to allow facile absorption.

Unlike prior art foamable compositions, the total surfactant employed to obtain a foam that is stable, of low specific gravity and has a fine bubble structure is low. Lower surfactant levels, particularly of ionic surfactants, are preferred to reduce skin irritations. Total surfactant is in the range of 0.1 to 5.0 wt% of the foamable composition, and is typically less than 2 wt%, or even less than 1 wt%.

### Water gelling agents

The water gelling agent according to one or more embodiments of the present invention stablizes the acqueons phase by, for example, increasing viscosity and linking capability. Exemplary water gelling agents that can be used in accordance with one or more embodiments of the present invention include for example, but are not limited to, naturally-occurring polymeric materials such as, locust bean gum, sodium alginate, sodium caseinate, egg albumin, gelatin agar, carrageenin gum sodium alginate, xanthan gum, quince seed extract, tragacanth gum, starch, chemically modified starches and the like, semi-synthetic polymeric materials such as cellulose ethers (e.g. hydroxyethyl cellulose, methyl cellulose, carboxymethyl cellulose, hydroxy propylmethyl cellulose), polyvinylpyrrolidone, polyvinylalcohol, guar gum, hydroxypropyl guar gum, soluble starch, cationic celluloses, cationic guars and the like and synthetic polymeric materials such as carboxyvinyl polymers, polyvinylpyrrolidone, polyvinyl alcohol polyacrylic acid polymers, polymethacrylic acid polymers, polyvinyl acetate polymers, polyvinyl chloride polymers, polyvinylidene chloride polymers and the like. Mixtures of the above compounds are contemplated.

Further exemplary water gelling agents include the acrylic acid/ethyl acrylate copolymers and the carboxyvinyl polymers sold, for example, by the B.F. Goodrich Company under the trademark of Carbopol Registered TM resins. These resins consist essentially of a colloidal water-soluble polyalkenyl polyether crosslinked polymer of acrylic acid crosslinked with from 0.75% to 2% of a crosslinking agent such as polyallyl sucrose or polyallyl pentaerythritol. Examples include Carbopol 934, Carbopol 940, Carbopol 950, Carbopol 980, Carbopol 951 and Carbopol 981. Carbopol 934 is a water-soluble polymer of acrylic acid crosslinked with about 1% of a polyallyl ether of sucrose having an average of about 5.8 allyl groups for each sucrose molecule.

The gelling agent is present in an amount in the range of about 0.1% to about 5.0 wt% of the foamable composition. In one or more embodiments, it is typically less than 1 wt% of the foamable composition.

### "Alcohol free"

Unlike the composition disclosed in US Pat. No. 6,126,920, which contains a 40-90 wt% aliphatic alcohol, the composition of the present invention does not contain such amount alcohols. For the purpose of the present application, the term "alcohol free" shall mean that the composition contains no more than an incidental amount of an aliphatic alcohol, e.g. less than about 7.5% of any aliphatic alcohol, having one to six carbon atoms in their carbon backbone, or no more than 7.5% of any mixture of such aliphatic alcohols. Alcohols at these low levels are not considered to have a negative effect on skin or mucous membranes. In one or more embodiments, the foamable compositions do not contain any alcohol.

### Optional Ingredients

The pharmaceutical or cosmetic foam carrier of the present invention may further optionally comprise a variety of pharmaceutical or cosmetic ingredients, which are added in order to fine-tune the consistency of the formulation, protect the formulation components from degradation and oxidation and bestow their cosmetic acceptability. Such excipients, may be selected, for example, from the group consisting of diglycerides, triglycerides, stabilizing agents, antioxidants, humectants, flavoring, colorant and odorant agents and other formulation components, used in the art of pharmaceutical and cosmetic formulary. A pharmaceutical or cosmetic composition manufactured using the foam carrier according to the present invention is very easy to use. When applied onto the afflicted body surface of humans or animals, it is in a foam state, allowing free application without spillage. Upon further application of a mechanical force, e.g., by rubbing the composition onto the body surface, it freely spreads on the surface and is rapidly absorbed.

### Propellant Aerosol

Aerosol propellants are used to generate and administer the foamable composition as a foam. The total composition including propellant, foamable compositions and optional ingredients is referred to as the foamable carrier. The propellant makes up about 5-25 wt% of the foamable carrier. Examples of suitable propellants include volatile hydrocarbons such as butane, propane, isobutane or mixtures thereof, and fluorocarbon gases.

### Composition and Foam Physical Characteristics

### 1. Composition flow properties:

It is important to have a composition, including water, hydrophobic solvents, formulation excipients and propellant, in a stable emulsion, which ascertain acceptable shelf-life of the product.

Yet, another crucial property is that said composition has to be free flowing, since otherwise, it cannot flow through the dip-tube of the aerosol container and create acceptable foam. It has been noted that in the context of the composition of the present invention, compositions comprising semi-solid hydrophobic solvents, e.g., white petrolatum, are excessively viscous and demonstrate poor flowability.

The combination of a surface active agent, foaming adjuvant and water gelling agent according to one or more embodiments of the invention provides a low specific gravity foam having superior flow properties and sheer breakability (among other attributes). According to one or more embodiments of the present invention, the total amount of surface active agent, foaming adjuvant and water gelling agent, in combination is less than 5 % (w/w) of foamable composition. The low solids content improves the flow properties of the foam, reduces unpleasant skin residue and reduces the cost of manufacture. As is demonstrated herein, the foam quality and foam breakability is excellent, despite the low levels of these components in the foam.

### 2. Foam properties:

The following scale for foam quality is used to evaluate foams.
**E** (excellent): very rich and creamy in appearance, does not show any bubble structure or shows a very fine (small) bubble structure.
**G** (good): rich and creamy in appearance, very small bubble size, "dulls" more rapidly than an excellent foam.
**FG** (fairly good): a moderate amount of creaminess noticeable, bubble structure is noticeable.
**F** (fair): very little creaminess noticeable, larger bubble structure than a "fairly good" foam.
**P** (poor): no creaminess noticeable, large bubble structure.
**VP** (very poor): dry foam, large very dull bubbles, difficult to spread on the skin.

Foams, adequate for topical administration according to the present invention have to be of quality grade E or G, upon release from the aerosol container. Smaller bubbles mean more stable foam, which does not collapse spontaneously immediately upon discharge from the container. The finer foam structure looks and feels smoother, thus increasing its usability and appeal.

A crucial aspect of foam properties, according to the present invention is breakability. Sheer-force breakability of the foam, as attained by the composition of the present invention is clearly advantageous to thermally-induced breakability, present, for example in US Pat. 6,126,920, and the respective Olux and Luxiq products, as demonstrated by the fact that according to the use instructions of Olux and Luxiq, the foam cannot be applied on the hand and afterwards delivered to the afflicted area, since it immediately collapses upon exposure to skin temperature.

Yet, another important property is specific gravity of the foam, as measured upon release from the aerosol can. Typically, foams according to the present invention have specific gravity of less than 0.1 g/mL and more preferably, less than 0.05 g/mL.

### FIELDS OF PHARMACEUTICAL APPLICATIONS

By including an appropriate therapeutic agent in the foamable carrier, the foam composition of the present invention is useful in the therapy of a variety of dermatological disorders (also termed "dermatoses"), including, in a non-limiting exemplary manner:
**Dermatitis**
   - Contact Dermatitis
   - Atopic Dermatitis
   - Seborrheic Dermatitis
   - Nummular Dermatitis
   - Chronic Dermatitis Of The Hands And Feet
   - Generalized Exfoliative Dermatitis
   - Stasis Dermatitis
   - Lichen Simplex Chronicus
**Bacterial Infections**
   - Cellulitis
   - Acute Lymphangitis
   - Lymphadenitis
   - Erysipelas
   - Cutaneous Abscesses
   - Necrotizing Subcutaneous Infections
   - Staphylococcal Scalded Skin Syndrome
   - Folliculitis
   - Furuncles
   - Hidradenitis Suppurativa
   - Carbuncles
   - Paronychial Infections
   - Erythrasma
**Fungal Infections**
   - Dermatophyte Infections
   - Yeast Infections
**Parasitic Infections**
   - Scabies
   - Pediculosis
   - Creeping Eruption
**Viral Infections**
**Disorders of Hair Follicles and Sebaceous Glands**
   - Acne
   - Rosacea
   - Perioral Dermatitis
   - Hypertrichosis (Hirsutism)
   - Alopecia, including male pattern baldness, alopecia areata, alopecia universalis and alopecia totalis
   - Pseudofolliculitis Barbae
   - Keratinous Cyst
**Scaling Papular Diseases**
   - Psoriasis
   - Pityriasis Rosea
   - Lichen Planus
   - Pityriasis Rubra Pilaris
**Benign Tumors**
   - Moles
   - Dysplastic Nevi
   - Skin Tags
   - Lipomas
   - Angiomas
   - Pyogenic Granuloma
   - Seborrheic Keratoses
   - Dermatofibroma
   - Keratoacanthoma
   - Keloid
**Malignant Tumors**
   - Basal Cell Carcinoma
   - Squamous Cell Carcinoma
   - Malignant Melanoma
   - Paget's Disease Of The Nipples
   - Kaposi's Sarcoma
**Reactions To Sunlight**
   - Sunburn
   - Chronic Effects of Sunlight
   - Photosensitivity
**Bullous Diseases**
   - Pemphigus
   - Bullous Pemphigoid
   - Dermatitis Herpetiformis
   - Linear Immunoglobulin A Disease
**Pigmentation Disorders**
   - Hypopigmentation
   - Vitiligo
   - Albinism
   - Postinflammatory hypopigmentation
   - Hyperpigmentation
   - Melasma (chloasma)
   - Drug-induced hyperpigmentation
   - Postinflammatory hyperpigmentation
**Disorders of Cornification**
   - Ichthyosis
   - Keratosis Pilaris
   - Calluses And Corns
   - Actinic keratosis
**Pressure Sores**
**Disorders of Sweating**
**Inflammatory reactions**
   - Drug Eruptions
   - Toxic Epidermal Necrolysis
   - Erythema Multiforme
   - Erythema Nodosum
   - Granuloma Annulare

In one or more embodiments of the present invention, the foam composition of the present invention is useful in the therapy of non-dermatological disorders, which respond to transdermal delivery of an active agent. By way of example, such disorders include localized pain in general, as well as joint pain, muscle pain, back pain, rheumatic pain, arthritis, ostheoarthritis and acute soft tissue injuries and sports injuries. Other disorders of this class include conditions, which respond to hormone therapy, such as hormone replacement therapy, transdermal nicotine administration, and other respective disorders, known in the art of drug delivery. The foam composition of the present invention is also useful in the delivery of local anesthetic agents.

### ACTIVE PHARMACEUTICAL AGENTS (DRUGS)

The active pharmaceutical agents, also referred to as "drug(s)", may consist of a single drug or a combination of drugs that can be dissolved in the water phase or the hydrophobic phase of the carrier composition. Examples of such drugs are antibiotic, antibacterial, antifungal, antiviral, antiinflammatory, anesthetic, analgesic, antiallergic, corticosteroid, retinoid and antiproliferative medications and mixtures thereof at any proportion. The concentration of drugs may be adopted to exert a therapeutic effect on a disease when applied to an afflicted area.

### Antibacterial agents

One important class of drugs comprises antibacterial agents. It is well known that bacterial infections are involved in a variety of superficial disorders of the skin, eye, mucosal membrane, oral cavity, vagina and rectum. The antibacterial drug can be active against gram positive and gram-negative bacteria, protozoa, aerobic bacteria and unaerobic ones.

By way of example, the antibacterial drugs can be selected from the group of chloramphenicol, tetracyclines, synthetic and semi-synthesic penicillins, beta-lactames, quinolones, fluoroquinolnes, macrolide antibiotics, metronidazlole and its derivatives and analogs, dicarboxylic acids, such as azelaic acid, slicylates, peptide antibiotics, cyclosporines and any combination thereof at a therapeutically effective concentration. Another group of antibacterial agents which is non-specific, comprises strong oxidants and free radical liberating compounds, such as hydrogen peroxide, bleaching agents (e.g., sodium, calcium or magnesium hypochloride and the like) iodine, chlorohexidine and benzoyl peroxide.

Antibacterial compositions according to the present invention may be used to treat infections of the skin. An example of a very common skin infection is acne, which involve infestation of the sebaceous gland with p. acnes, as well staphylococus aurus and pseudomonas. Various antibacterial agents have been utilized to treat acne, however, their efficacy is limited due to their low penetration into the hydrophobic environment of the skin layers and sebaceous glands. The composition of the present invention, comprising a hydrophobic component, would facilitate an enhanced rate of penetration. Furthermore, the intrinsic antibacterial and antiinflammatory effects of the foam adjuvant agents, i.e., fatty alcohols and acids, provides a combined effect that should result in a better therapeutic response to treatment.

The composition of the present invention is particularly useful and beneficial in the prevention and treatment of secondary infections, accompanying skin-structure damage, such as in cuts, wounds, burns and ulcers. In all such cases, the present formulation is easy to use, being in foam state when applied and becoming liquid instantly upon rubbing onto the skin.

While being useful in the prevention and treatment of infections, the antibacterial foam of the present invention is also applicable for decontaminating areas, afflicted with bacterial warfare organisms, such as anthrax and smallpox.

The same advantage is expected when the composition of the present invention is topically applied to mucosal membranes, the oral cavity, the vagina and the rectum.

### Anti-fungal agents

Fungal infections are another object of treatment using the composition of the present invention. Superficial fungal infection of the skin is one of the commonest skin diseases seen in general practice. Dermatophytosis is probably the most common superficial fungal infection of the skin. It is caused by a group of fungi, which are capable of metabolizing the keratin of human epidermis, nails or hair. There are 3 genera of dermatophytes causing dermatophytosis, i.e, microsporum, trichophyton and epidermophyton.

Candidiasis is an infection caused by the yeast like fungus candida albicans or occasionally other species of candida. Clinical syndromes of candidiasis include: (a) oral candidiasis (oral thrush); (b) candidiasis of the skin and genital mucous membrane; and (c) candida paronychia, which inflicts the nail.

The pharmaceutical composition may comprise an antifungal drug, which is active against dermatophytes and candida, selected from the group of, but not limited to azoles, diazoles, triazoles, miconazole, fluconazole, ketoconazole, clotrimazole, itraconazole griseofulvin; ciclopirox, amorolfine, terbinafine, Amphotericin B, potassium iodide, flucytosine (5FC) and any combination thereof at a therapeutically effective concentration.

It is useful, for example for the treatment of tinea corporis, tinea pedis, tinea rubrum, tinea unguium, tinea cruris, tinea barbae and tinea versicolor, as well as yeast Infections, such as candidiasis, and candidal vaginitis

### Anti-viral agents

The composition of the present invention is particularly beneficial in the case of viral infections. Cold sores are caused by the herpes simplex Type 1 virus and are sometimes referred to as facial herpes. Mollusca are small viral growths that appear singly or in groups on the face, trunk, lower abdomen, pelvis, inner thighs, or penis. Shingles (herpes zoster), which usually only occurs once in a lifetime, appears as a rash (clusters of blisters with a red base). It is caused by the same virus responsible for chickenpox. Warts are a common, benign skin tumor caused by viral infection.

Viral infections are currently treated with various antiviral agents, as summarized in the following table:

| **Drug** | **Viruses** | **Chemical Type** |
|---|---|---|
| Vidarabine | Herpesviruses | Nucleoside analogue |
| Acyclovir | Herpes simplex (HSV) | Nucleoside analogue |
| Gancyclovir | Cytomegalovirus (CMV) | Nucleoside analogue |
| Nucleoside-analog reverse transcriptase inhibitors (NRTI): AZT (Zidovudine), ddl (Didanosine), ddC (Zalcitabine), d4T (Stavudine), 3TC (Lamivudine) | Retroviruses (HIV) | Nucleoside analogue |
| Non-nucleoside reverse transcriptase inhibitors (NNRTI): Nevirapine, Delavirdine | Retroviruses (HIV) | Nucleoside analogue |
| Protease Inhibitors: Saquinavir, Ritonavir, Indinavir, Nelfinavir | HIV | Peptide analogue |
| Ribavirin | Broad spectrum: HCV, HSV, measles, mumps, Lassa fever | Triazole carboxamide |
| Amantadine / Rimantadine | Influenza A strains | Tricyclic amine |
| Interferons | Hepatitis B and C | Protein |

Any of the above antiviral drugs, in a therapeutically effective concentration, can be incorporated in the foam composition of the present invention. The composition of the present invention, which comprises a hydrophobic solvent, would facilitate an enhanced rate of penetration and better topical distribution of any of the above listed antiviral drugs. Furthermore, the intrinsic antiviral effects of the foam adjuvant agents, i.e., fatty alcohols and acids, provides a combined effect that should result in a better therapeutic response to treatment.

### Antiinflammatory or antiallergic agents

Yet, according to another embodiment according to the present invention the drug is an antiinflammatory or antiallergic agent. Antiinflammatory or antiallergic agent can be selected from the group of corticosteroids, non-steroidal antiinflammatory drugs (NSAIDs), anti-histamines, immunosuppressants and any combination thereof at a therapeutically effective concentration.

The following table provides a summary of currently available corticosteroid agent and their typical therapeutically effective concentration.

| **Potency** | **Compound** | **Formulation** |
|---|---|---|
| Very high | Clobetasol proprionate | Cream or ointment 0.05% |
| | Halobetasol proprionate | Cream or ointment 0.05% |
| High | Betamethasone diproprionate | Cream or ointment 0.05% |
| | Betamethasone valerate | Ointment 0.1% |
| | Fluocinolone acetonide | Cream 0.02% |
| | Halcinonide | Cream or ointment 0.1 % |
| Medium | Betamethasone valerate | Cream 0.1% |
| | Fluocinolone acetonide | Cream or ointment 0.020% |
| | Hydrocortisone valerate | Cream or ointment 0.2% |
| | Triamcinolone acetonide | Cream, ointment, or lotion 0.1 % or 0.020% |
| Low | Hydrocortisone | Cream, ointment, or lotion 1.0% or 2.5% |

The concentrations of corticosteroid drugs, as presented in the above table are provided herein only as example, and any therapeutically effective concentration of such corticosteroids can be incorporated in the composition of the present invention.

Since all corticosteroid drugs are typically hydrophobic, the carrier of the present invention, comprising a hydrophobic solvent, is most suitable as a vehicle to facilitate better topical distribution and an enhanced rate of penetration of any of the above listed drugs. Furthermore, the intrinsic antiviral, antibacterial and antiinflammatory effects of the foam adjuvant agents, i.e., fatty alcohols and acids, provides a combined effect that should result in a better therapeutic response to treatment.

Psoriasis is a very common chronic skin disease, which may be the target of treatment using the composition of the present invention. It is marked by periodic flare-ups of sharply defined red patches covered by a silvery, flaky surface.

Corticosteroid ointments, greasy preparations containing little or no water, are commonly used for treating psoriasis. Their main disadvantage is in their sticky feeling, which remains so long after treatment is over. By contrast, the foam of the present invention, while comprising considerable concentration of an oil (hydrophobic solvent), spreads very easily throughout the afflicted area and absorbs into the skin without leaving any untoward sensation or look. Examples of other inflammatory disorders, which can be treated by the composition of the present invention, wherein the drug is a steroid are atopic dermatitis, seborrhea, seborrheic dermatitis of the face and trunk, seborrheic blepharitis, contact dermatitis, stasis dermatitis (gravitational eczema; varicose eczema), exfoliative dermatitis (erythroderma), lichen simplex chronicus, pityriasis rosea and pemphigus.

Topical antihistaminic preparations currently available include 1% and 2% diphenhydramine (Benadryl^{®} and Caladryl^{®}), 5% doxepin (Zonalon^{®}) cream, phrilamine maleate, chlorpheniramine and tripelennamine, phenothiazines, promethazine hydrochloride (Phenergan^{®}) and dimethindene maleate. These drugs, as well as additional antihistamins can also be incorporated in the composition of the present invention.

It is pointed out that polyunsaturated fatty acids, containing omega-3 and omega-6 fatty acids (e.g., linoleic and linolenic acid, gamma-linoleic acid (GLA), eicosapentaenoic acid (EPA) and docosahexaenoic acid (DHA) are beneficial in the treatment of psoriasis and other skin inflammation conditions.

A second class of anti-inflammatory agents, which is useful in the foam of the present invention, includes the nonsteroidal anti-inflammatory agents (NSAIDs). The variety of compounds encompassed by this group is well-known to those skilled in the art. Specific non-steroidal anti-inflammatory agents useful in the composition invention include, but are not limited to:
1) Oxicams, such as piroxicam, isoxicam, tenoxicam, sudoxicam;
2) Salicylates, such as salicylic acid, ethyl salicylate, methyl salycilate, aspirin, disalcid, benorylate, trilisate, safapryn, solprin, diflunisal, and fendosal;
3) Acetic acid derivatives, such as diclofenac, fenclofenac, indomethacin, sulindac, tolmetin, isoxepac, furofenac, tiopinac, zidometacin, acematacin, fentiazac, zomepirac, clindanac, oxepinac, felbinac, and ketorolac;
4) Fenamates, such as mefenamic, meclofenamic, flufenamic, niflumic, and tolfenamic acids;
5) Propionic acid derivatives, such as ibuprofen, naproxen, benoxaprofen, flurbiprofen, ketoprofen, fenoprofen, fenbufen, indopropfen, pirprofen, carprofen, oxaprozin, pranoprofen, miroprofen, tioxaprofen, suprofen, alminoprofen, and tiaprofenic; and
6) Pyrazoles, such as phenylbutazone, oxyphenbutazone, feprazone, azapropazone, and trimethazone.

Any further steroidal and nonsteroidal compounds, having the capacity to prevent, alleviate the symptoms of, treat or cure inflammation processes, are generally included, as possible anti-inflammatory agents, according to the present invention.

The pharmaceutical composition of the present invention may also comprise an antiinflammatory or antiallergic agent, wherein said agent reduces the occurrence of pro-inflammatory cytokines or inhibits the effect of pro-inflammatory cytokines.

Mixtures of such anti-inflammatory agents may also be employed, as well as the dermatologically acceptable salts, esters, amides, prodrugs and derivatives of these agents.

Topical application of a foam, comprising a safe and effective dose of an NSAID can be useful in the prevention and/or alleviation of the symptoms of rheumatoid arthritis, osteoarthritis and pain. Topical NSAIDs, incorporated in the foam of the present invention can be also used in the treatment of dermatological disorders, such as acne, rosacea, hair growth disorders, actinic keratosis and certain skin cancer conditions.

### Topical Anesthetics

The compositions of the present invention may contain a safe and effective amount of a topical anesthetic. Examples of topical anesthetic drugs include benzocaine, lidocaine, bupivacaine, chlorprocaine, dibucaine, etidocaine, mepivacaine, tetracaine, dyclonine, hexylcaine, procaine, cocaine, ketamine, pramoxine, phenol, and pharmaceutically acceptable salts thereof. Mixtures of such anesthetic agents may be synergistically beneficial.

### Keratolytically active agents

The term "keratolytically active agent" is used herein to mean a compound which loosens and removes the stratum corneum of the skin, or alters the structure of the keratin layers of skin.

Keratolytically active agents are used in the treatment of many dermatological disorders, which involve dry skin, hyperkeratiinization (such as prsoriasis), skin itching (such as xerosis), acne and rosacea.

Suitable keratolytically active agent include phenol and substituted phenolic compounds. Such compounds are known to dissolve and loosen the intracellular matrix of the hyperkeratinized tissue. As such, they are used in the treatment of dermatological disorders. Dihydroxy benzene and derivatives thereof have been recognized as potent keratolytic agents. Resorcinol ( m-dihydroxybenzene) and derivatives thereof are used in anti-acne preparations. Hydroquinone (p-dihydroxybenzene), besides its anti-pigmentation properties, is also keratolytic. These compounds also exhibit antiseptic properties. Cresols also possess bactericidal and keratolytic properties.

Vitamin A and its derivatives, such as retinoic acid, isoretinoic acid, retinol and retinal are another preferred class of keratolytically active agents.

Another group of keratolytically active agents include alpha-hydroxy acids, such as lactic acid and glycolic acid and their respective salts and derivatives; and beta-hydroxy acids, such as Salicylic acid (o-hydroxybenzoic acid) and its salts and pharmaceutically acceptable derivatives, which typically possess anti-inflammatory, as well as keratolytic, activity.

Yet, another class of preferred keratolytically active agents includes urea and its derivatives.

### Retinoids

Another preferred group of active agents comprise retinol, retinal, all trans retinoic acid and derivatives, isomers and analogs thereof, collectively termed "retinoids". Etretinate, actiretin, isotretinoin, adapalene and tazarotene are further examples of said retinoid isomers and analogs. Compositions according to the present invention, which contain retinoids as the active drug, can be used for the treatment of acne, seborrhea, various dermatoses, inflammation of the skin, mucosal membranes, vagina and the rectum, psoriasis, actinic keratosis and skin cancers, by application onto the affected area.

### Insecticide and Insect repellents agents

Insects, such as mosquitoes, biting flies, mites, gnats, fleas, chiggers, punkies, sand flies, lice and ticks can be annoying and sometimes pose a serious risk to human and animal health. In certain areas of the United States, mosquitoes can transmit diseases like equine and St. Louis encephalitis. Biting flies can inflict a painful bite that can persist for days, swell, and become infected. Ticks can transmit serious diseases like Lyme disease and Rocky Mountain spotted fever.

There are several types of insect repellents to use when protecting people and animals from flying or biting insects, spiders, ticks and mites. By way of example, these may include DEET (N, N-diethyl-m-toluamide), dimethyl phthalate, piperonyl butoxide and permethrin. Insect repelling terpenoids, have been reported by Hwang, et al, J. Chem. Ecol., 11, 1297 (1985); and Ruledge, J. Am. Mosquito Control Assoc. 4, 414 (1988).

A particularly preferred group of insect repellents includes the terpenoid compounds, described in U.S. Patent No. 5,411,992, including:
(1) Terpenoid-alcohol or terpene-ols are terpenoids which have at least one hydroxyl group. Examples of terpene-ols include: C₁₀H₁₆O compounds, perillyl alcohol, carveol, myrtenol, and cis-verbenol; C₁₀H₁₈O compounds, myrtanol, iso-pinocampheol, dihydrocarveol, isopulegol, terpineol, terpinen-4-ol, nerol, geraniol, and linalool, and C₁₀H₂₀O compounds, menthol, beta-citronellol, and dihydro-myrcenol.
(2) Terpenoid-esters are terpenoids, which have at least one ester group which is the product of the bonding of the hydroxyl group of a terpene-ol with an aliphatic carboxylic acid that can contain functional groups such as the hydroxyl or amine on the aliphatic chain. Examples of suitable aliphatic carboxylic acids include acetic acid, propionic acid, lactic acid, and various amino acids. Examples of terpenoid-esters include: carvyl acetate, carvyl propionate, and menthyl lactate.
(3) Essential oils which contain terpenoids and perfumes which contain terpenoids. Non-limiting examples of essential oils which have high content of terpene-ols and esters include bergamot (62% terpenoids); sage (>50% terpenoids); styrax (>50% terpenoids); peppermint (>50% terpenoids); and pine Siberian (75% terpenoids %). Terpenes, aldehydes and ketones vary in their usefulness but as a general group have potential as insect-repellent.

The foam of the present invention is particularly suitable for the effective uniform spreading of an insect repellent agent onto large areas of the skin of humans and animals. The hydrophobic solvent present in the foam composition helps retain the insect repellent on the skin surface for an extended period of time.

Yet, in a further embodiment, the foam is suitable for delivery of insect-killing agents (insecticides) to an afflicted external surface area of humans and animals. Thus, the pharmaceutical or cosmetic composition may comprise an insecticide, known in the art of parasitology. By way of example, such insecticide can be selected selected from the group of permethrin, hexachlorobenzene, carbamate, naturally occuring pyrethroids, permethrin, allethrin, malathion, piperonyl butoxide and any combination thereof at a therapeutically effective concentration. Its application is very convenient and it spreads easily, even over hairy areas. The hydrophobic solvent present in the foam composition helps retain the insecticide on the treated area for an extended period of time. Furthermore, the presence of a hydrophobic solvent in the foam eases mechanical removal of lice and nits with a comb.

### Anti cancer drugs

Anti cancer drugs can also be used according to the present invention as the drug of choice from skin malignant tumors, such as basal cell carcinoma, squamous sell carcinoma, melanoma and Kaposi's sarcoma, as well as the pre-cancerous condition actinic keratosis. In certain cases, topical cytotoxic and antiproliferative drugs are used to treat or prevent such cancers, including 5-fluorouracil, also called 5-FU. 5-FU, as well as any other anti-cancer agents, know in the art of cancer medicine, can be incorporated in the foam at therapeutically effective levels.

A preferred family of anticancer drugs, suitable for usage in the foam of the present formulation comprises antiestrogens, such as tamoxifen. Tamoxifen blocks the effects of the hormone estrogen in the body. It is used to prevent or delay the return of breast cancer or to control its spread.

### Photodynamic therapy agents

The foam composition of the present invention is also useful to deliver photo-sensitizing agents, known in the art of photodynamic therapy. By way of example, such photosensitizers can be selected from the group comprising modified porphyrins, chlorins, bacteriochlorins, phthalocyanines, naphthalocyanines, pheophorbides, purpurins, m-THPC, mono-L-aspartyl chlorin e6, bacteriochlorins, phthalocyanines, benzoporphyrin derivatives, as well as photosensitiser precursors, such as aminolevulinic acid (ALA).

### Active agents for burns, wounds, cuts and ulcers

The treatment of burns, wounds, cuts and ulcers, using the composition of the present invention is particularly advantageous. The foam can include both anti-infective agents (against bacteria, fungi and/or viruses), antiinflammatory agents (steroidal and/or NSAIDs) and pain relieving components. Upon application, the foam spreads easily, covering the surface of the affected area, and without causing pain.

### SKIN CARE ACTIVE AGENTS

The foam of the present invention is useful and advantageous for skin care and cosmetic care. The combination of oil and water, having moisture-retaining properties, in a spreadable foam form, can be used to substitute currently used cosmetic skin care creams, lotions, gels, etc. The cosmetic foam compositions of the present invention are suitable for the further application as "cosmeceutical" preparation (cosmetic products with therapeutic benefit), to treat "cosmetic" skin disorders, such as aging skin, wrinkles, hyperpigmentation (melasma, chloasma, freckles, etc.), scaly skin and other skin undesirable properties.

The CTFA Cosmetic Ingredient Handbook describes a wide variety of nonlimiting cosmetic and pharmaceutical ingredients commonly used in the skin care industry, which are suitable for use in the compositions of the present invention. Examples of these ingredient classes include: abrasives, absorbents, aesthetic components such as fragrances, pigments, colorings/colorants, essential oils, astringents, etc. (e.g., clove oil, menthol, camphor, eucalyptus oil, eugenol, menthyl lactate, witch hazel distillate), anti-acne agents, antimicrobial agents (e.g., iodopropyl butylcarbamate), antioxidants, binders, biological additives, buffering agents, bulking agents, chelating agents, chemical additives, colorants, cosmetic astringents, cosmetic biocides, denaturants, drug astringents, external analgesics, film formers or materials, e.g., polymers, for aiding the film-forming properties and substantivity of the composition (e.g., copolymer of eicosene and vinyl pyrrolidone), opacifying agents, pH adjusters, propellants, reducing agents, sequestrants, skin bleaching and lightening agents (e.g., hydroquinone, kojic acid, ascorbic acid, magnesium ascorbyl phosphate, ascorbyl glucosamine), skin-conditioning agents (e.g., humectants, including miscellaneous and occlusive), skin soothing and/or healing agents (e.g., panthenol and derivatives (e.g., ethyl panthenol), aloe vera, pantothenic acid and its derivatives, allantoin, bisabolol, and dipotassium glycyrrhizinate), skin treating agents, thickeners, and vitamins and derivatives thereof.

In any embodiment of the present invention, however, the active agents useful herein can be categorized by the benefit they provide or by their postulated mode of action. It is to be understood that the active agents useful herein can in some instances provide more than one benefit or operate via more than one mode of action. Therefore, classifications herein are made for the sake of convenience and are not intended to limit the active to that particular application or applications listed.

### Anti-acne active agents

The compositions of the present invention may contain a safe and effective amount of one or more pharmaceutically or cosmetically acceptable anti-acne active agents. Examples of useful anti-acne actives include resorcinol, sulfur, salicylic acid and salicylates, alpha-hydroxy acids, nonsteroidal anti-inflammatory agents, benzoyl peroxide, retinoic acid, isoretinoic acid and other retinoid compounds, adapalene, tazarotene, azelaic acid and azelaic acid derivatives, antibiotic agents, such as erythromycin and clyndamycin, zinc salts and complexes, and combinations thereof, in a therapeutically effective concentration.

### Anti-wrinkle active agents/anti-atrophy active agents and agents to treat dry and scaly skin (xerosis and ichthyosis)

The compositions of the present invention may further contain a safe and effective amount of one or more anti-wrinkle actives or anti-atrophy actives, which can be easily delivered by spreading a foam onto the skin. Exemplary anti-wrinkle/anti-atrophy active agents suitable for use in the compositions of the present invention include sulfur-containing D and L amino acids and their derivatives and salts, particularly the N-acetyl derivatives; thiols; hydroxy acids (e.g., alpha-hydroxy acids such as lactic acid and glycolic acid and their derivatives and salts; or beta-hydroxy acids such as salicylic acid and salicylic acid salts and derivatives), urea, hyaluronic acid, phytic acid, lipoic acid; lysophosphatidic acid, skin peel agents (e.g., phenol, resorcinol and the like), vitamin B3 compounds (e.g., niacinamide, nicotinic acid and nicotinic acid salts and esters, including non-vasodilating esters of nicotinic acid (such as tocopheryl nicotinate), nicotinyl amino acids, nicotinyl alcohol esters of carboxylic acids, nicotinic acid N-oxide and niacinamide N-oxide), vitamin B5 and retinoids (e.g., retinol, retinal, retinoic acid, retinyl acetate, retinyl palmitate, retinyl ascorbate). In the case of dry, scaly skin (xerosis) and ichthyosis such agents can alleviate the symptoms by temporary relief of itching associated with these conditions.

### Anti-oxidants/radical scavengers

A safe and effective amount of an anti-oxidant/radical scavenger may be added to the compositions of the subject invention, preferably from about 0.1 % to about 10% (w/w), more preferably from about 1% to about 5% (w/w), of the composition.

Anti-oxidants/radical scavengers such as ascorbic acid (vitamin C) and its salts, ascorbyl esters of fatty acids, ascorbic acid derivatives (e.g., magnesium ascorbyl phosphate, sodium ascorbyl phosphate, ascorbyl sorbate), tocopherol (vitamin E), tocopherol sorbate, tocopherol acetate, other esters of tocopherol, butylated hydroxy benzoic acids and their salts, 6-hydroxy-2,5,7,8-tetramethylchroman-2-carboxylic acid (commercially available under the tradename Trolox.sup.R), gallic acid and its alkyl esters, especially propyl gallate, uric acid and its salts and alkyl esters, sorbic acid and its salts, lipoic acid, amines (e.g., N,N-diethylhydroxylamine, amino-guanidine), sulfhydryl compounds (e.g., glutathione), dihydroxy fumaric acid and its salts, lycine pidolate, arginine pilolate, nordihydroguaiaretic acid, bioflavonoids, curcumin, lysine, methionine, proline, superoxide dismutase, silymarin, tea extracts, grape skin/seed extracts, melanin, and rosemary extracts may be used.

The foam of the present invention is suitable for delivering skin protecting and revitalizing anti-oxidants/radical scavengers. It is further pointed out that polyunsaturated fatty acids, containing omega-3 and omega-6 fatty acids (e.g., linoleic and linolenic acid, gamma-linoleic acid (GLA), eicosapentaenoic acid (EPA) and docosahexaenoic acid (DHA) are beneficial in the treatment of psoriasis and other skin inflammation conditions. Likewise, emollients and silicone oils exert moisture-retaining and skin protective effects on the skin. Thus in a preferred embodiment, a skin protective foam is provided, wherein the hydrophobic solvent comprises in full or in part, a solvent, selected from the group of emollients, silicone oil and oils, rich in unsaturated fatty acids, thus, affording a synergistic therapeutic effect of the anti-oxidants/radical scavenger agent and the vehicle components.

### Self-tanning active agents

The foam of the present invention is particularly suitable for the uniform delivery of a tanning active agent onto large areas of the skin. It is preferable that the compositions contain from about 0.1% to about 20%, more preferably from about 2% to about 7%, and still more preferably from about 3% to about 6%, of the composition, of dihydroxyacetone, or any other compound, know in the art as an artificial tanning active agent.

### Skin Lightening and Whitening Agents

The foam of the present invention is particularly suitable for the uniform delivery of a skin lightening agent. When used, the compositions preferably contain from about 0.1% to about 10%, more preferably from about 0.2% to about 5%, of the composition, of a skin-lightening agent. Suitable skin lightening or whitening agents include those known in the art, including hydroquinone, azelaic acid and other related dicarboxylic acids, and salts and derivatives thereof, retinoids, kojic acid, arbutin, nicotinic acid and its precursors, salts and derivatives, arbutin, ascorbic acid and salts and derivatives thereof (e.g., magnesium ascorbyl phosphate or sodium ascorbyl phosphate), and herbal extracts (e.g., licorice extract, mulberry extract, placental extract).

In one or more embodiments of the present invention, the foam composition comprises a combination of a skin whitening agent and a sunscreen agent.

In one or more embodiments of the present invention, the foam composition comprises a combination of a skin whitening agent and an inorganic sunscreen agent. When inorganic sunscreen agents, e.g. TiO₂, are rubbed onto the skin, they leave a white coating, which provides an immediate (although transient) whitening effect, which is highly desirable by the consumer, who wishes to see instant change in his/her appearance. The whitening agent, in combination with the inorganic sunscreen agent in the foam carrier can be easily and uniformly distributed on the skin surface, thereby affording an even instant whitening effect, unlike creams that are difficult to spread evenly on skin areas.

### Sunscreens

Exposure to ultraviolet light can result in excessive scaling and texture changes of the stratum corneum. The foam of the present invention is advantageous for the delivery of sunscreen agents. Its application is very convenient and it spreads easily over large skin areas. The presence of a hydrophobic solvent in the foam ensures long lasting effect, even while bathing.

As used herein, *"sunscreen* active" or "sunscreen agent" includes both sunscreen agents and physical sunblocks. Suitable *sunscreen* actives may be organic or inorganic.

Inorganic sunscreens useful herein include the following metallic oxides; titanium dioxide having an average primary particle size of from about 15 nm to about 100 nm, zinc oxide having an average primary particle size of from about 15 nm to about 150 nm, zirconium oxide having an average primary particle size of from about 15 nm to about 150 nm, iron oxide having an average primary particle size of from about 15 nm to about 500 nm, and mixtures thereof. When used herein, the inorganic sunscreens are present in the amount of from about 0.1 % to about 20%, preferably from about 0.5% to about 10%, more preferably from about 1% to about 5%, of the composition.

A wide variety of conventional organic sunscreen actives are suitable for use herein. Specific suitable sunscreen actives include, for example: p-aminobenzoic acid, its salts and its derivatives (ethyl, isobutyl, glyceryl esters; p-dimethylaminobenzoic acid); anthranilates (i.e., o-amino-benzoates; methyl, menthyl, phenyl, benzyl, phenylethyl, linalyl, terpinyl, and cyclohexenyl esters); salicylates (amyl, phenyl, octyl, benzyl, menthyl, glyceryl, and di-pro-pyleneglycol esters); cinnamic acid derivatives (menthyl and benzyl esters, a-phenyl cinnamonitrile; butyl cinnamoyl pyruvate); dihydroxycinnamic acid derivatives (umbelliferone, methylumbelliferone, methylaceto-umbelliferone); trihydroxy-cinnamic acid derivatives (esculetin, methylesculetin, daphnetin, and the glucosides, esculin and daphnin); hydrocarbons (diphenylbutadiene, stilbene); dibenzalacetone and benzalacetophenone; naphtholsulfonates (sodium salts of 2-naphthol-3,6-disulfonic and of 2-naphthol-6,8-disulfonic acids); dihydroxynaphthoic acid and its salts; o- and p-hydroxybiphenyldisulfonates; coumarin derivatives (7-hydroxy, 7-methyl, 3-phenyl); diazoles (2-acetyl-3-bromoindazole, phenyl benzoxazole, methyl naphthoxazole, various aryl benzothiazoles); quinine salts (bisulfate, sulfate, chloride, oleate, and tannate); quinoline derivatives (8-hydroxyquinoline salts, 2-phenylquinoline); hydroxy- or methoxy-substituted benzophenones; uric and violuric acids; tannic acid and its derivatives (e.g., hexaethylether); (butyl carbotol) (6-propyl piperonyl) ether; hydroquinone; benzophenones (oxybenzene, sulisobenzone, dioxybenzone, benzoresorcinol, 2,2',4,4'-tetrahydroxybenzophenone, 2,2'-dihydroxy-4,4'-dimethoxybenzophenone, octabenzone; 4-isopropyldibenzoylmethane; butylmethoxydibenzoylmethane; etocrylene; octocrylene; [3-(4'-methylbenzylidene bornan-2-one), terephthalylidene dicamphor sulfonic acid and 4-isopropyl-di-benzoylmethane.

A safe and effective amount of the organic sunscreen active is used, typically from about 1% to about 20%, more typically from about 2% to about 10% of the composition. Exact amounts will vary depending upon the sunscreen or sunscreens chosen and the desired Sun Protection Factor (SPF).

### Agents for Hair Growth Disorders

Agents, which affect the pattern of hair growth, can be suitably incorporated in the foam of the present invention. Male patterrn baldness (MPB), the commonest cause of balding, is induced by the activity of the male hormone dihydrotestosterone (DHT), which converted from the hormone testosterone by the enzymes 5 alpha reductase. Current treatments of MPB include minoxidil and agents, which inhibit 5 alpha reductase, such as finasteride, spironolactone, azelaic acid and azelaic acid derivatives and salts. Such agents, as well as other agents known in the art, can be incorporated in the foam of the present invention.

It is further pointed out that polyunsaturated fatty acids, i.e., such which include any of the essential fatty acids (EFA's): linoleic and linolenic acid, gamma-linoleic acid (GLA), eicosapentaenoic acid (EPA) and docosahexaenoic acid (DHA), are also known to contribute to hair growth. Thus in a preferred embodiment, a hair growth foam is provided, wherein the hydrophobic solvent comprises in full or in part, an oil, rich in such unsaturated fatty acids.

### Figure-forming Agents; Agents to Treat Cellulite / Slimming

Figure forming agents such as used in the treatment of cellulite and in slimming products, can be suitably incorporated in the foam of the present invention. A non-limiting exemplary list of active agents, known in the treatment of cellulite and in the induction of a slimming effect include herbal extracts, such as baldderwack extract, butcher's, broom, cayenne, dandelion, red clover, ginkgo biloba, horse chestnut, witch hazel and borage oil, omega 3 and omega 6 oils, caffeic acid and salts and derivatives thereof, xanthine agents, such as caffeine, theophiline and pentoxyphilline, and nicotinic acid and salts and derivatives thereof.

### Agents to Treat Sunburn, Heat Burn, Radiation Burn, Rash and Itch

Cosmetic and pharmaceutical ingredients which are known in the art of pharmacology and cosmetology to treat dermatitis, minor skin irritations, sunburn, heat burn, radiation burn, and inhibit inflammation can be beneficially incorporated in the foam of the present invention.

Examples of such active agents include chamomile extract (matricaria recutitia), cucumber distillate (cucumis sativus), lavender water (lavendula angustifolia), rose water (rosa damascena), witch hazel (hamamelis virginiana), allantoin, bisabolol, rosehip oil, calendula oil, azulaene, menthol and camphor.

### Use of the Foam as a Lubricating and Protective Foam

There are several potential uses of the foam, particularly the silicone-oil based foam, as a lubricating foam. Typical examples are shaving foam, moisture protection foam and antifriction foam. For such purposes, the foam can be used in its basic composition (without additional formulation aids and active ingredients), or with the addition of such additives.

### FOAM FOR NEUTRALIZATION AND/OR DECONTAMINATION OF HAZARDOUS CHEMICALS AND TREATMENT OF HEAT BURNS

It has been reported that povidone iodine antiseptic, a popular iodine product, can ameliorate damage to guinea pig skin exposed to mustard gas and other chemical irritants and further reduces, and many times prevents, damage to human skin after accidental heat burns caused by hot water, oil or hot steam.

Other active compound, having decontamination abilities, comprise strong oxidants and free radical liberating compounds, such as hydrogen oxide, bleaching agents (e.g., sodium, calcium or magnesium hypochloride and the like) iodine, chlorohexidine and benzoyl peroxide.

The alcohol-free foam of the present invention, comprising one or more of the above decontaminating and neutralizing agents can be applied onto the contaminated skin to form a preventive layer, prior to contamination measure or as a decontamination/neutralization means, right after contamination has occurred.

### PENETRATION ENHANCERS

A penetration enhancer or permeation enhancer is an agent used to increase the permeability of the skin to a pharmacologically active agent to increase the rate at which the drug diffuses through the skin and enters the tissues and bloodstream. A chemical skin penetration enhancer increases skin permeability by reversibly altering the physiochemical nature of the stratum corneum to reduce its diffusional resistance. In a review of the technical and patent literature up to 1996, numerous chemical compounds were cited as skin penetration enhancers. Most of the compounds are generally recognized as safe (GRAS) ingredients that would often be considered inert by a formulator (Osborne D W, Henke J J, Pharmaceutical Technology, November 1997, pp 58-86.)

Examples of penetration enhancers, according to the present invention include: polyols, such as propylene glycol, hexylene glycol, diethylene glycol, propylene glycol n-alkanols, terpenes, di-terpenes, tri-terpenes, terpen-ols, limonene, terpene-ol, 1-menthol, dioxolane, ethylene glycol, other glycols, and glycerol; sulfoxides, such as dimethylsulfoxide (DMSO), dimethylformanide, methyl dodecyl sulfoxide, dimethylacetamide; monooleate of ethoxylated glycerides (with 8 to 10 ethylene oxide units); Azone (1-dodecylazacycloheptan-2-one), 2-(n-nonyl)-1,3-dioxolane; esters, such as isopropyl myristate/palmitate, ethyl acetate, butyl acetate, methyl proprionate, capric/caprylic triglycerides, octylmyristate, dodecyl-myristate; myristyl alcohol, lauryl alcohol, lauric acid, lauryl lactate ketones; amides, such as acetamide oleates such as triolein; various surfactants, such as sodium lauryl sulfate; various alkanoic acids such as caprylic acid; lactam compounds, such as azone; alkanols, such as oleyl alcohol; dialkylamino acetates, and admixtures thereof.

Lower alcohols, such as ethanol, propanol, isopropanol, butanol, isobutanol, t-butanol and pentanol are not considered appropriate penentartion enhancers according to the present invention, due to their skin drying and irritation properties.

Yet, another preferred class of penetration enhancers in the cyclodextrines and related compounds. Cyclodextrins are structurally related cyclic oligomaltoses which form a new group of pharmaceutical excipients. These are torus-shaped molecules with a hydrophilic outer surface and a lipophilic central cavity. Cyclodextrins are capable of forming water-soluble inclusion complexes with a wide variety of lipophilic water-insoluble drugs by taking up a whole drug molecule, or some part of it, into the cavity. The cyclodextrin molecules are relatively large (molecular weight ranging from almost 1000 to over 1500), with a hydrated outer surface, and under normal conditions, cyclodextrin molecules will only permeate the skin barrier with considerable difficulty. It is generally believed that the cyclodextrin molecules act as true carriers by keeping lipophilic drug molecules in solution and deliver them to the skin surface where they partition from the cyclodextrin cavity into the skin.

### FURTHER TECHNICAL PARAMETERS

The composition of the present invention may be contained in and dispensed from a container capable of withstanding the pressure of the propellant gas and having an appropriate valve/nozzle for dispensing the composition as foam under pressure. A customary liquefied propellant can be added, in the amount of about 5-25% of the total composition. Liquefied propellants are gases that exist as liquids under pressure, including high purity hydrocarbons such as propane, isobutane and n-butane, dimethyl ether and chlorofluorocarbons (CFCs).

A specific embodiment according to the present invention comprises placing the composition of the present invention on a patch, occlusive tape or the skin-contact compartment of a transdermal delivery apparatus and applying such object onto the skin, in order to attain effective superficial treatment or enhanced penetration of the drug into the skin or through the skin.

Utilizing such strategy, one can apply drugs, which are currently administered systemically or that require transdermal delivery, in the preferred therapeutic system of the present invention. Examples for such drugs are nicotine, testosterone and other male hormones and male hormone precursors, estrogen and other female hormones and hormone precursors, growth hormone, insulin, caffeine, steroidal and non-steroidal antiinflammatory agents and thyroid hormone substitutes.

The general process, as typically exemplified in Example 1 may be applied in order to produce the composition of the present invention.
The pharmaceutical carrier according to the present invention can also be used to prepare cosmetics for beauty purpose by adding into skin care agents and perfume.

### EXAMPLES

The invention is described with reference to the following examples. This invention is not limited to these examples and experiments. Many variations will suggest themselves and are within the full intended scope of the appended claims.

### Example 1 - General Procedure for Preparing Foamable Composition.

Aqueous Phase: Water gelling agent and surface-active agent are dissolved in water, with agitation. The solution is warmed to 50-70°C. Water soluble cosmetic or pharmaceutical active ingredients and optional water soluble ingredients are added with agitation to the Aqueous Phase mixture.

Hydrophobic Phase: The hydrophobic solvent is heated to same temperature. Foam adjuvant agent is added to preheated hydrophobic solvent. Oil soluble cosmetic or pharmaceutical active ingredients* and optional oil soluble formulation ingredients are added with agitation to the Hydrophobic Phase mixture.

The warm Hydrophobic Phase is gradually poured into the warm Aqueous Phase, with agitation, followed by Ultraturax homogenization. The mixture is allowed to cool down to ambient temperature. In case of heat sensitive active ingredients, the active ingredient is added with agitation to the mixture after cooling to ambient temperature. The mixture, at ambient temperature, is added to an aerosol container, the container is sealed and appropriate amount of propellant (5-25 w% of the composition mass) is added under pressure into the container.

### Example 2 - Vegetable Oil-Based Foam Carrier Composition

| | **Ingredient** | **Version No. 1** | **Version No. 2** | **Version No. 3** |
|---|---|---|---|---|
| | | % (W/W) | | |
| **Hydrophobic solvent** | Soybean oil | 40 | 30.5 | 20 |
| **Water** | Water | 48.5 | 32.5 | 61 |
| **Foam adjuvant agent** | Stearyl Alcohol | 0.8 | 1.05 | 0.73 |
| **Surface-active agent** | Sucrose ester SP70 | 0.64 | 0.45 | 0.8 |
| **Water gelling agent** | Xanthan Gum | 0.16 | 0.11 | 0.1 |
| | Methocel ELV15 | 0.32 | 0.22 | 0.28 |
| **Other Ingredients** | Antioxidant | 0.02 | 0.02 | 0.02 |
| | Preservatives | 0.3 | 0.3 | 0.3 |
| | Fragrance | 0.2 | 0.2 | 0.2 |
| | | | | |
| **Foam Specific gravity (gr/mL)** | | **0.10** | **0.15** | **0.065** |

The compositions use a non-ionic surfactant and contain a combined amount of surface-active agent, foam adjuvant and water gelling agent ranging from 1.83 % to 1.92 % (w/w). The foam of this example is useful as a carrier of active pharmaceutical and/or cosmetic active ingredients, as exemplified below. It also can be used as a protective product. Additionally, it is also useful as lubricating foam, for various purposes.

### Example 3 - Silicone Oil-Based Foam Carrier Composition

| | **Specific Ingredient** | **Version No.1** | **Version No. 2** |
|---|---|---|---|
| | | % (W/W) | |
| **Hydrophobic solvent** | Dimeticone 350* | 25 | 10 |
| **Water** | Water | 72 | 87 |
| **Foam adjuvant agent** | Stearyl Alcohol | 0.2 | 0.2 |
| **Surface-active agent** | Sucrose ester SP70 | 0.8 | - |
| | Myrj 49P | - | 0.8 |
| **Water gelling agent** | Xanthan Gum | 0.2 | 0.2 |
| | Methocel ELV15 | 0.4 | 0.4 |
| **Other Ingredients** | Antioxidant | 0.02 | 0.02 |
| | Preservatives | 1 | 1 |
| | Fragrance | 0.2 | 0.2 |
| | | | |
| **Foam Specific gravity (gr/mL)** | | **0.10** | **ND** |
| * Dimethylpolysiloxane of 350 cps viscosity. | | | |

The compositions use only non-ionic surfactant and contain a combined amount of surface-active agent, foam adjuvant and water gelling agent of 1.6% (w/w). The foam of this example is useful as a carrier of active pharmaceutical and/or cosmetic active ingredients, as exemplified below. It also can be used as a protective product. Additionally, it is also useful as lubricating foam, for various purposes.

### Example 6 - Mineral Oil-Based Foam Carrier Composition

| | **Ingredient** | **Version No. 1** | **Version No.2** | **Version No. 3** | **Version No. 4** | **Version No. 5** |
|---|---|---|---|---|---|---|
| | | %(W/W) | | | | |
| **Hydrophobic solvent** | Mineral oil | 69 | 50 | 50 | 25 | 25 |
| **Water** | Water | 28.4 | 46.7 | 46.7 | 71.88 | 71.9 |
| **Foam adjuvant agent** | Stearyl Alcohol | 0.7 | 1 | 1 | 0.5 | 0.5 |
| **Surface-active agent** | Sucrose ester SP70 | 0.4 | 0.64 | 0 | 0.8 | 0 |
| | PEG S-40 | 0 | 0 | 0.64 | 0 | 0 |
| | Polysorbate-60 | 0 | 0 | 0 | 0 | 0.8 |
| **Water gelling agent** | Xanthan Gum | 0.1 | 0 | 0.14 | 0.2 | 0.2 |
| | Methocel ELV15 | 0.2 | 0.4 | 0.32 | 0.4 | 0.4 |
| **Other Ingredients** | Antioxidant | 0.02 | 0.02 | 0.02 | 0.02 | 0.02 |
| | Preservatives | 1 | 1 | 1 | 1 | 1 |
| | Fragrance | 0.2 | 0.2 | 0.2 | 0.2 | 0.2 |
| | | | | | | |
| **Foam Specific gravity (gr/mL)** | | **ND** | **ND** | **ND** | **ND** | **0.1** |

The compositions use only non-ionic solvents, and the total amount of surface active agent, foam adjuvants and water gelling agents ranges from 1.4 to 2.1 % (w/w). The foam of this example is useful as a carrier of active pharmaceutical and/or cosmetic active ingredients, as exemplified in examples below. It is also useful as lubricating foam, for various purposes.

### Example 7 - Mixed Oils Foam Carrier Composition

| | **Ingredient** | Version No. 1 25% Oil | Version No. 2 12.5% Oil |
|---|---|---|---|
| **Hydrophobic solvent** | Mineral oil | 11.2% | 5.6% |
| | Isopropyl myristate | 5.0% | 2.5% |
| | MCT oil | 7.5% | 3.8% |
| **Foam adjuvant agent** | Stearyl Alcohol | 0.5% | 0.25% |
| **Water** | Water | 73.0% | 85.2% |
| **Surface-active agent** | Sucrose ester SP70 | 0.8% | 0.8% |
| | | | |
| | Distilled monoglyceride | 1.2% | 0.6% |
| | Sodium lauryl sulphate | 0.1% | 0.1% |
| **Water gelling agent** | Xanthan Gum | 0.3% | 0.3% |
| | Methocel ELV15 | 0.6% | 0.6% |

The foams of this example have a non-ionic surfactant to ionic surfactant ratio (w/w) of 20:1 and 14:1 for versions 1 and 2, respectively. Total amounts of surface active agent foam adjuvant and water gelling agent is in the range of 1.75 - 3.5 % (w/w). It is useful as a carrier of active pharmaceutical and/or cosmetic active ingredients, as exemplified in examples below. It is also useful as lubricating foam, for various purposes.

The following examples, representing optional drug-containing foams, are prototype formulations, which have not been optimized for stability and inter-component compatibility. Such optimization is a customary need, which can be done, using means, known to those skilled in the art of pharmaceutical formulation

### Example 8 - Antibacterial Foam Composition

| **Ingredient** | **Version 1 "Mupirocin"** | **Version 2 "Triple Antibiotic"** | **Version 3 "Fucidic Acid"** | **Version 4 "Metronidazole"** | **Version 5 "Triple Antibiotic"** |
|---|---|---|---|---|---|
| **Carrier Ingredients** | | | | | |
| Mineral oil | 48.8% | 11.2% | 48.8% | 5.6% | 5.6% |
| Isopropyl myristate | | 5.0% | | 2.5% | 2.5% |
| MCT oil | | 7.5% | | 3.8% | 3.8% |
| Stearyl Alcohol | 0.8% | 0.5% | 0.8% | 0.25% | 0.25% |
| Water | 50% | 73.0% | 50% | 85.2% | 85.2% |
| Sucrose ester SP70 | 0.8% | 0.8% | - | | 0.8% |
| Myrj 40 | - | | 0.8% | - | - |
| Distilled monoglyceride | | 1.2% | | 0.6% | 0.6% |
| Tween 60 | | | | 0.8% | |
| Sodium lauryl sulphate | 0.05% | 0.1% | 0.1% | | |
| Xanthan Gum | 0.2% | 0.3% | 0.2% | 0.3% | 0.3% |
| **Active Ingredients** | | | | | |
| Mupirocin | 2% | | | | |
| Polymyxin B Sulfate | | 10,000 Units/gr | | | 10,000 Units/gr |
| Bacitracin Zinc | | 500 Units/gr | | | 500 Units/gr |
| Neomycin Sulfate* | | 0.05% | | | 0.05% |
| Pramoxine HCl | | 1% | | | 1% |
| Fucidic acid | | | 2% | | |
| Metronidazole | | | | 1% | |
| Methocel ELV15 | 0.2% | 0.6% | 0.2% | 0.6% | 0.6% |

The foams of this example contain 100% non-ionic surfactant or have a non-ionic surfactant to ionic surfactant ratio ranging from 20:1 to 8:1. Total amounts of surface active agent, foam adjuvant and water gelling agent ranges from 2.05 - 3.5 % (w/w). It is useful for the treatment of bacterial skin infection (general), cellulites, open wounds, cutaneous abscesses, furuncles, insect bite, impetigo, acne, acne-rosacea, and trichomonas vaginitis.

In certain embodiments, the foam of this example is useful for the prevention, decontamination and/or neutralization hazardous bacterial infestation (such as warfare organisms).

### Example 9 - Antifungal Foam Composition

| **Ingredient** | **Version 1 "Terbinafine"** | **Version 2 "Clotrimazole"** | **Version 3 "Nystatin"** | **Version 4 "Nystatin"** |
|---|---|---|---|---|
| **Carrier Ingredients** | | | | |
| Mineral oil | 48.8% | 11.2% | 48.8% | 5.6% |
| Isopropyl myristate | | 5.0% | | 2.5% |
| MCT oil | | 7.5% | | 3.8% |
| Stearyl Alcohol | 0.8% | 0.5% | 0.8% | 0.25% |
| Water | 50% | 73.0% | 50% | 85.2% |
| Sucrose ester SP70 | 0.8% | 0.8% | - | 0.8% |
| Myrj 40 | - | | 0.8% | - |
| Tween 80 | | | | 0.8% |
| Distilled monoglyceride | | 1.2% | | 0.6% |
| Sodium lauryl sulphate | 0.05% | 0.1% | 0.1% | |
| Xanthan Gum | 0.2% | 0.3% | 0.2% | 0.3% |
| **Active Ingredients** | | | | |
| Terbinafine | 1% | | | |
| clotrimazole | | 2% | | |
| Nystatin | | | 100,000 Units/gr | 100,000 Units/gr |
| Methocel ELV15 | 0.2% | 0.6% | 0.2% | 0.6% |

The foams of this example have 100% non-ionic surfactant or have a non-ionic surfactant to ionic surfactant ratio ranging from 20:1 to 8:1. Total surface active agent, foaming adjuvant and water gelling agent ranges from 2.05 to 3.5% (w/w). It is useful in the treatment of dermatophyte infections, Tinea corporis, Tinea pedis, Tinea rubrum, Tinea unguium, Tinea cruris, Tinea barbae, and yeast infections, such as Candidiasis, Tinea versicolor and Candidal vaginitis.

### Example 10 - Corticosteroid Foam Composition

| **Ingredient** | **Version 1 "Hydrocortisone"** | **Version 2 "Betamethasone"** | **Version 3 "Dexamethasone"** |
|---|---|---|---|
| **Carrier Ingredients** | | | |
| Mineral oil | 48.8% | 11.2% | 5.6% |
| Isopropyl myristate | | 5.0% | 2.5% |
| MCT oil | | 7.5% | 3.8% |
| Stearyl Alcohol | 0.8% | 0.5% | 0.25% |
| Water | 50% | 73.0% | 85.2% |
| Sucrose ester SP70 | 0.8% | 0.4% | 0.8% |
| Tween 80 | | 0.4% | |
| Distilled monoglyceride | | 1.2% | 0.6% |
| Sodium lauryl sulphate | 0.05% | | 0.1% |
| Xanthan Gum | 0.2% | 0.3% | 0.3% |
| Methocel ELV15 | 0.2% | 0.6% | 0.6% |
| **Active Ingredients** | | | |
| Hydrocortisone | 1% | | |
| Betamethasone dipropionate | | 0.05% | |
| Dexamethasone acetate | | | 0.1% |
| | | | |

| **Ingredient** | **Version 4 "Triamcinolone"** | **Version 5 "Flumetasone"** | |
|---|---|---|---|
| **Carrier Ingredients** | | | |
| Mineral oil | 48.8% | 48.8% | |
| Stearyl Alcohol | 0.8% | 0.8% | |
| Water | 50% | 50% | |
| Sucrose ester SP70 | 0.8% | 0.8% | |
| Sodium lauryl sulphate | 0.05% | 0.05% | |
| Xanthan Gum | 0.2% | 0.2% | |
| **Active Ingredients** | | | |
| Triamcinolone acetonide | 0.1% | | |
| Flumetasone pivalate | | 0.02% | |
| Methocel ELV15 | 0.2% | 0.2% | |

The foams of this example have either 100% non-ionic surfactant or have a non-ionic surfactant to ionic surfactant ratio ranging from 20:1 to 16:1. Total surface active agent, foaming adjuvant and water gelling agent ranges from 2.05 to 3.5% (w/w). Indications include psoriasis, contact dermatitis, atopic dermatitis, seborrheic dermatitis, nummular dermatitis, inflammatory acne, chronic dermatitis of the hands and feet, generalized exfoliative dermatitis, stasis dermatitis, lichen simplex chronicus, herpes gestationis and pruritic urticarial papules and plaques of pregnancy.

### Example 11 - Antiviral Foam Composition

| **Ingredient** | **Version 1 "Acyclovir"** | **Version 2 "Acyclovir"** | **Version 3 "α-Interferon**" |
|---|---|---|---|
| **Carrier Ingredients** | | | |
| Mineral oil | 48.8% | 11.2% | 5.6% |
| Isopropyl myristate | | 5.0% | 2.5% |
| MCT oil | | 7.5% | 3.8% |
| Stearyl Alcohol | 0.8% | 0.5% | 0.25% |
| Water | 50% | 73.0% | 85.2% |
| Sucrose ester SP70 | | 0.8% | 0.8% |
| Tween 80 | 0.8% | | |
| Distilled monoglyceride | | 1.2% | 0.6% |
| Sodium lauryl sulphate | | | 0.1% |
| Xanthan Gum | 0.2% | 0.3% | 0.3% |
| **Active Ingredients** | | | |
| Acyclovir | 5% | 5% | |
| α-Interferon | | | 105 IU/g |
| Methocel ELV15 | 0.2% | 0.6% | 0.6% |

The foams of this example have either 100% non-ionic surfactant or have a non-ionic surfactant to ionic surfactant ratio of 14:1. Total surface active agent, foaming adjuvant and water gelling agent ranges from 2.05 to 3.5% (w/w). Indications include Herpes simplex, Herpes zoster, Herpes gestationis and Herpes simplex genital ulcers.

### Example 12 - Insect repellent Foam Composition

| **Ingredient** | **%** |
|---|---|
| Isopropyl myristate | 2.0% |
| MCT oil | 2.0% |
| Stearyl Alcohol | 1.2% |
| Water | 64.0% |
| Sucrose ester SP70 | 0.8% |
| Sodium lauryl sulphate | 0.1% |
| Xanthan Gum | 0.3% |
| Methocel ELV15 | 0.6% |
| Propylene glycol | 15% |
| DEET | 15% |

### Example 14 - Comparative Tolerability and Acceptability Study of a Corticosteroid Foam Composition Vs. a Conventional Ointment

A panel of eight testers was requested to apply about 0.5 gr. of the foam preparation of example 10, Version 2 on one arm and 0.5 gr. of commercial Betamethasone valerate ointment, in a double blind fashion. They were asked to describe their feeling about the ease of application, ease of spreading, spreadability and penetrability of each of the products and to give their general rating for each of the products on a scale of 0-3 (0 = poor; 1=barely acceptable; 2=acceptable and 3=excellent).

As demonstrated in the following table, the foam preparation of example 10, Version 2 obtained higher rates in all aspects of the test.

| **Property** | **Foam Preparation Mean Rating** | **Commercial Betamethasone Valerate Ointment Mean Rating** |
|---|---|---|
| Ease of application | 2.3 | 1.6 |
| Ease of spreading | 2.5 | 1.9 |
| Spreadability | 2.9 | 1.2 |
| Penetrability | 2.0 | 1.5 |
| Lack of sticky feeling | 2.4 | 1.0 |
| Lack of greasy feeling | 2.2 | 1.0 |
| Lack of shiny look | 1.9 | 1.4 |
| Overall rating | 2.5 | 1.4 |

### Example 15: Human Safety and Efficacy Study of a Corticosteroid Composition in psoriasis patients

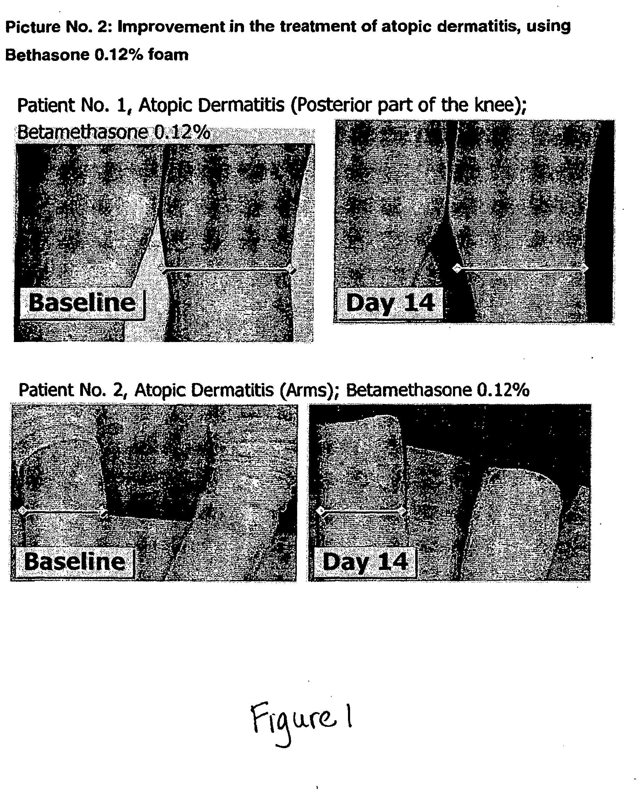

Two patients with mild to moderate psoriasis were administered topically a Betamethasone 0.12% foam (example 10, Version 2) twice daily for two weeks. Both patients improved significantly, as manifested by clearance of the psoriatic plaques flattening of the thickened lesions. Figure 1 provides an exemplary response to treatment in the elbows of one of these patients. While betamethasone is know for its effect in psoriasis, such a beneficial effect after 14 days treatment is exceptional. The accelerated effect was attributed to the improved convenience and therefore, improved compliance.

### Example 16: Human Safety and Efficacy Study of a Corticosteroid Composition in psoriasis patients

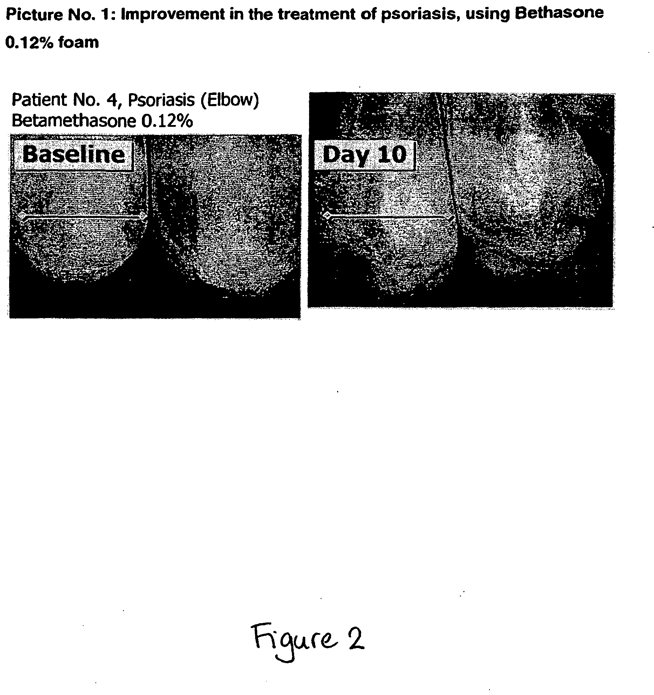

Four patients with moderate to severe, disseminated atopic dermatitis were administered topically a Betamethasone 0.12% foam (example 10, Version 2) twice daily for two weeks. All patients improved significantly, as manifested by complete clearance of all treated lesions. Figure 2 provides exemplary responses to treatment in different body areas, after 10 days of treatment. While betamethasone is know for its effect in atopic dermatitis, such a beneficial effect after 10 days treatment is exceptional. The patients claimed that the use of the foam of the present invention was significantly more convenient than the corresponding cream and ointment. Thus, the accelerated effect was attributed to the improved convenience and therefore, improved compliance.

### Example 17 - Foam Compositions with Urea

| **Component** | **% w/w** | | | |
|---|---|---|---|---|
| Mineral oil | 6.00 | 6.00 | 6.00 | 6.00 |
| Isopropylmeristat | 6.00 | 6.00 | 6.00 | 6.00 |
| Glyceryl monostearate | 0.50 | 0.50 | 0.50 | 0.50 |
| Stearyl alcohol | 0.20 | 0.20 | 0.20 | 1.00 |
| Urea | 10.00 | 10.00 | 10.00 | 10.00 |
| Xantan gum | 0.30 | 0.30 | 0.30 | 0.30 |
| Methocel K100M | 0.30 | 0.30 | 0.30 | 0.30 |
| Myrj 52 | | | | 3.00 |
| TWEEN 80 | | | | 1.00 |
| Myrj 49p | | | 3.00 | |
| TWEEN 60 | 1.00 | 1.00 | 1.00 | |
| Cocamidopropylbetain | 0.50 | 0.50 | | |
| Phenonip | 0.30 | 0.30 | 0.30 | 0.30 |
| Water | to 100.0 | to 100.0 | to 100.0 | to 100.0 |
| | | | | |
| Butane/propane | 8.00 | 8.00 | 18.00 | 18.00 |
| | | | | |
| FoamQuality | E | E | E | E |
| Density | n/a | 0.023 | n/a | 0.024 |

### Example 18 - Compositions with various penetration enhancers

| **Component** | **% w/w** | | | | |
|---|---|---|---|---|---|
| Mineral oil | 6.00 | 6.00 | 6.00 | 6.00 | 6.00 |
| Isopropyl myristate | 6.00 | 6.00 | 6.00 | 6.00 | 6.00 |
| Glyceryl monostearate | 0.50 | 0.50 | 0.50 | 0.50 | 0.50 |
| Stearyl alcohol | 1.00 | 1.00 | 1.00 | 1.00 | 1.00 |
| Xantan gum | 0.30 | 0.30 | 0.30 | 0.30 | 0.30 |
| Methocel K100M | 0.30 | 0.30 | 0.30 | 0.30 | 0.30 |
| TWEEN 60 | 1.00 | | | | |
| TWEEN 80 | | 1.00 | 1.00 | 1.00 | 1.00 |
| MYRJ 49p | 3.00 | 3.00 | 3.00 | 3.00 | 3.00 |
| Propylen glycol | | **5.00** | | | |
| Glycofurol | | | **1.00** | **10.00** | |
| Urea | | | | | **10.00** |
| Cocoamidopropylbethaine | 0.50 | 0.50 | 0.50 | 0.50 | 0.50 |
| Lidocain base | 4.00 | 4.00 | 4.00 | 4.00 | 4.00 |
| Phenonip | 0.30 | 0.30 | 0.30 | 0.30 | 0.30 |
| Water | to 100 | to 100 | to 100 | to 100 | to 100 |
| | | | | | |
| Butane/propane | 8 | 8 | 8 | 16 | 10 |
| | | | | | |
| Foam Quality | E | E | E | E | E |
| Density | 0.020 | 0.018 | 0.019 | 0.019 | 0.018 |

| **Component** | **% w/w** | **%w/w** | | | |
|---|---|---|---|---|---|
| Isopropyl myristate | 30.00 | 30.00 | | | |
| Glyceryl monostearate | 0.50 | 0.50 | | | |
| Stearic acid | 0.45 | 0.45 | | | |
| Xantan gum | 0.30 | 0.30 | | | |
| Methocel K100M | 0.30 | 0.30 | | | |
| TWEEN 80 | 1.00 | 1.00 | | | |
| MYRJ 49p | 3.00 | 3.00 | | | |
| Cocoamidopropylbethaine | 0.50 | 0.50 | | | |
| Transcutol p | 20.00 | 20.00 | | | |
| Hydrophilic drug | Effective concentration | | | | |
| Hydrophobic drug | | Effective concentration | | | |
| Phenonip | 0.30 | 0.30 | | | |
| Water | to 100.0 | to 100.0 | | | |
| | | | | | |
| Butane/propane | 8.00 | 8.00 | | | |
| | | | | | |
| FoamQuality | E | E | | | |
| Density | 0.020 | 0.020 | | | |

## Claims

1. An alcohol-free foamable pharmaceutical or cosmetic carrier, comprising:
a foamable composition comprising:
about 2-5% by weight of composition of a liquid, non-volatile hydrophobic solvent and about 80-98% by weight of composition of water; or about 5-10% by weight of composition of a liquid, non-volatile hydrophobic solvent and about 75-95% by weight of composition of water; or about 10-20% by weight of composition of a liquid, non-volatile hydrophobic solvent and about 60-90% by weight of composition of water; or about 20-75% by weight of composition of a liquid, non-volatile hydrophobic solvent and about 25-75% by weight of composition of water;
about 0.1% to 5% by weight of composition of a foam adjuvant agent selected from the group consisting of fatty alcohols, fatty acids, hydroxyl-substituted fatty alcohols, hydroxyl-substituted fatty acids, and fatty acids and fatty alcohols including at least one double bond in its carbon atom chain;
about 0.1% to 5% by weight of composition of a surfactant, wherein the surfactant is solely non-ionic, comprising one or more non-ionic surfactants, or wherein the surfactant is a mixture of one or more non-ionic surfactants and one or more ionic surfactants in a ratio greater than 6:1, and
about 0.1% to 5% by weight of composition of a water gelling agent,
and
a liquefied propellant, at a concentration of about 5% to about 25% by weight of the foamable carrier, wherein the liquefied propellant comprises a volatile hydrocarbon or fluorocarbon gas;
wherein, the combined amount of foam adjuvant agent, surface-active agent and water gelling agent is less than about 5% by weight of the foamable composition;
wherein when the composition is released from a container, it provides a shear-force breakable foam suitable for topical or mucosal administration that does not break down easily on discharge but which upon rubbing onto the skin collapses easily.

2. The foamable carrier of claim 1, wherein the hydrophobic solvent comprises
about 5-10% by weight of composition, or
about 10-20% by weight of composition, or
about 20-75% by weight of composition.

3. The foamable carrier of any preceding claim, wherein the hydrophobic solvent comprises a mixture of mineral oil and an emollient in a ratio between 2:8 and 8:2 on a weight basis.

4. The foamable carrier of any preceding claim, wherein at least 2% of the foamable composition is a silicone oil.

5. The foamable carrier of any preceding claim, wherein the ionic surfactant is an, anionic surfactant, cationic surfactant, amphoteric or zwitterionic surfactant.

6. The foamable carrier of any preceding claim, wherein the surface-active agent is a mixture of a non-ionic surfactant and an anionic surfactant in a 20:1 to 1:1 ratio, or the surface-active agent is a mixture of a non-ionic surfactant and an anionic surfactant in a 100:1 to 6:1 ratio.

7. The foamable carrier of any preceding claim, wherein the surface-active agent has HLB value of more than 9.

8. The foamable carrier of any preceding claim, wherein the surfactant comprises a sucrose ester.

9. The foamable carrier of any preceding claim, wherein the hydrophobic solvent is selected from the group comprising vegetable oils, marine oils, mineral oils, emollients, silicone oils, plant-derived therapeutic oils and any mixture thereof at any proportion.

10. A pharmaceutical or cosmetic composition, comprising:
a foamable carrier as claimed in any preceding claim; and
an active agent, wherein where the composition is a pharmaceutical composition, the active agent is in a therapeutically effective amount.

11. The pharmaceutical or cosmetic composition of claim 10, wherein the active agent is a drug or a cosmetically effective agent.

12. The pharmaceutical or cosmetic composition of claim 10 or 11, further comprising excipients selected from the group consisting of antioxidants, humectants, flavoring, colorant and odorant agents.

13. The composition of any of claims 10 to 12, further comprising an effective concentration of a penetration enhancer.

14. The pharmaceutical or cosmetic composition of any of claims 10 to 13 wherein the active agent is selected for the treatment of a disease, the etiology of which is bacterial, fungal, viral, parasitic, inflammatory, autoimmune, allergic, hormonal, malignant and combinations thereof; wherein the active agent is selected for the treatment of a superficial condition; wherein the active agent is selected for the treatment of a disorder of the skin, mucosal membrane, vagina or rectum; wherein the active agent is selected for the treatment of a disorder, selected from the group of dermatosis, dermatitis, bacterial Infections, fungal Infections, parasitic infections, viral infections, disorders of hair follicles and sebaceous glands, acne, rosacea, scaling papular diseases, benign tumors, malignant tumors, reactions to sunlight, bullous diseases, pigmentation disorders, disorders of cornification, pressure sores, disorders of sweating, inflammatory reactions, xerosis, ichthyosis, allergy, burn, wound, cut, and non-dermatological disorders, which respond to transdermal delivery of said active agent; wherein the active agent is selected for the treatment of wounds, burns, cuts and ulcers; wherein the active agent is antibacterial, antifungal or antiviral; wherein the active agent is an insecticide or an insect repellent; wherein the active agent is an anti-inflammatory or antiallergic agent; wherein the active agent is an anticancer agent, a photodynamic therapy agent, a local anesthetic, a nonsteroidal anti-inflammatory drug (NSAID); or intended for transdermal delivery; wherein the active agent is a retinoid, an anti-wrinkle agent, or a skin-whitening agent; wherein said active agent is selected from the group comprising sulphur-containing amino acids, thiol compounds, alpha hydroxy acids, lactic acid and its derivatives and salts, glycolic acid and its derivatives and salts, beta-hydroxy acids, salicylic acid and salicylic acid salts and derivatives, phytic acid, lipoic acid, lysophosphatidic acid, skin peel agents, phenol, resorcinol, vitamin B3 compounds, niacinamide, nicotinic acid and nicotinic acid salts and esters, tocopheryl nicotinate, nicotinyl amino acids, nicotinyl alcohol esters of carboxylic acids, nicotinic acid N-oxide and niacinamide N-oxide, retinoids, retinol, retinal, retinoic acid, retinyl acetate, retinyl palmitate, retinyl ascorbate, caffeine, theophiline, pentoxyphilline, dihydroxy acetone kojic acid, arbutin, nicotinic acid and its precursors, salts and derivatives, arbutin, ascorbic acid and salts and derivatives thereof; wherein the active agent is an herbal extract, a radical scavenger, a self-tanning agent, an anti-acne active agent, a figure forming agent, an agent that influences hair growth, a hair growth stimulating agent, a hair growth inhibiting agent, a sunscreen agent, an inorganic sunscreen agent, a combination of a skin whitening agent and a sunscreen agent, or a combination of a skin whitening agent and an inorganic sunscreen agent.

15. The composition of any of claims 10 to 14, further comprising a decontaminating agent selected from the group comprising an oxidizing agent, iodine and iodine compounds, chlorohexidine, bleaching agents and surface-active agents.

16. The composition as claimed in any of claims 10 to 15 for use in a method of treatment of the human or animal body by therapy.

17. The use of a composition as claimed in any of claims 10 to 16 for the manufacture of a medicament for the treatment of a disease, the etiology of which is bacterial, fungal, viral, parasitic, inflammatory, autoimmune, allergic, hormonal, malignant and combinations thereof; or for the treatment of dermatosis, dermatitis, bacterial Infections, fungal Infections, parasitic infections, viral infections, disorders of hair follicles and sebaceous glands, scaling papular diseases, benign tumors, malignant tumors, reactions to sunlight, bullous diseases, pigmentation disorders, disorders of cornification, pressure sores, disorders of sweating, inflammatory reactions, xerosis, ichthyosis, allergy, burn, wound, cut, and non-dermatological disorders, which respond to transdermal delivery of said active agent.

18. A method of cosmetic treatment comprising administering topically a composition as claimed in any of claims, wherein the composition is a cosmetic composition and wherein the treatment is not a therapeutic treatment.

## Patentansprüche

1. Alkoholfreier, schäumbarer pharmazeutischer oder kosmetischer Träger, umfassend:
eine schäumbare Zusammensetzung, umfassend:
etwa 2-5 Gew.-% der Zusammensetzung eines flüssigen, nicht flüchtigen, hydrophoben Lösungsmittels und etwa 80-98 Gew.-% Wasser; oder etwa 5-10 Gew.-% der Zusammensetzung eines flüssigen, nicht flüchtigen, hydrophoben Lösungsmittels und etwa 75-95 Gew.-% Wasser; oder etwa 10-20 Gew.-% der Zusammensetzung eines flüssigen, nicht flüchtigen, hydrophoben Lösungsmittels und etwa 60-90 Gew.-% Wasser; oder etwa 20-75 Gew.-% der Zusammensetzung eines flüssigen, nicht flüchtigen, hydrophoben Lösungsmittels und etwa 25-75 Gew.-% Wasser;
etwa 0,1 bis 5 Gew.-% der Zusammensetzung eines Schaumhilfsstoffs, der ausgewählt ist aus der Gruppe bestehend aus Fettalkoholen, Fettsäuren, Hydroxyl-substituierten Fettalkoholen, Hydroxyl-substituierten Fettsäuren und Fettsäuren und Fettalkoholen, die mindestens eine Doppelbindung in ihrer Kohlenstoffatomkette aufweisen;
etwa 0,1 bis 5 Gew.-% der Zusammensetzung eines Tensids, wobei das Tensid ausschließlich nichtionisch ist, umfassend ein oder mehrere nichtionische Tenside, oder wobei das Tensid eine Mischung aus einem oder mehreren nichtionischen Tensiden und einem oder mehreren ionischen Tensiden in einem größeren Verhältnis als 6:1 ist; und
etwa 0,1 bis 5 Gew.-% der Zusammensetzung eines Wassergelbildners; und
ein verflüssigtes Treibmittel mit einer Konzentration von etwa 5 bis etwa 25 Gew.-% des schäumbaren Trägers, wobei das verflüssigte Treibmittel ein Kohlenwasserstoff- oder Fluorkohlenstoffgas umfasst;
wobei die kombinierte Menge des Schaumhilfsstoffs, des Tensids und des Wassergelbildners weniger als etwa 5 Gew.-% der schäumbaren Zusammensetzung beträgt;
wobei, wenn die Zusammensetzung aus einem Behälter freigegeben wird, sie einen durch Scherkraft zerbrechbaren Schaum für die topische oder mucosale Verabreichung bereitstellt, der bei der Ausgabe nicht leicht zerfällt, der jedoch beim Reiben auf die Haut leicht zusammenfällt.

2. Schäumbarer Träger nach Anspruch 1, wobei das hydrophobe Lösungsmittel folgendes umfasst:
etwa 5 bis 10 Gew.-% der Zusammensetzung; oder etwa 10 bis 20 Gew.-% der Zusammensetzung; oder etwa 20 bis 75 Gew.-% der Zusammensetzung.

3. Schäumbarer Träger nach einem der vorstehenden Ansprüche, wobei das hydrophobe Lösungsmittel eine Mischung als Mineralöl und einem Weichmacher in einem Verhältnis zwischen 2:8 und 8:2 auf Basis des Gewichts umfasst.

4. Schäumbarer Träger nach einem der vorstehenden Ansprüche, wobei mindestens 2% der schäumbaren Zusammensetzung ein Silikonöl sind.

5. Schäumbarer Träger nach einem der vorstehenden Ansprüche, wobei das ionische Tensid ein anionisches Tensid, ein kationisches Tensid, ein amphoterisches oder zwitterionisches Tensid ist.

6. Schäumbarer Träger nach einem der vorstehenden Ansprüche, wobei das Tensid eine Mischung aus einem nicht ionischen Tensid und einem anionischen Tensid in einem Verhältnis von 20:1 bis 1: ist, oder wobei das Tensid eine Mischung eines nicht ionischen Tensids und eines anionischen Tensids in einem Verhältnis von 100:1 bis 6:1 ist.

7. Schäumbarer Träger nach einem der vorstehenden Ansprüche, wobei das Tensid einen HLB-Wert von über 9 aufweist.

8. Schäumbarer Träger nach einem der vorstehenden Ansprüche, wobei das Tensid einen Saccharoseester umfasst.

9. Schäumbarer Träger nach einem der vorstehenden Ansprüche, wobei das hydrophobe Lösungsmittel ausgewählt ist aus der Gruppe umfassend Pflanzenöle, Meeresöle, Mineralöle, Weichmacher, Silikonöle, aus Pflanzen gewonnenen therapeutischen Ölen und einer Mischung dieser in jedem beliebigen Verhältnis.

10. Pharmazeutische oder kosmetische Zusammensetzung, umfassend:
einen schäumbaren Träger nach einem der vorstehenden Ansprüche; und
einen Wirkstoff, wobei die Zusammensetzung eine pharmazeutische Zusammensetzung ist, wobei der Wirkstoff in einer therapeutisch wirksamen Menge vorhanden ist.

11. Pharmazeutische oder kosmetische Zusammensetzung nach Anspruch 10, wobei der Wirkstoff ein Arzneimittel oder ein kosmetischer Wirkstoff ist.

12. Pharmazeutische oder kosmetische Zusammensetzung nach Anspruch 10 oder 11, ferner umfassend Arzneiträger, die aus der Gruppe ausgewählt sind bestehend aus Antioxidationsmitteln, Befeuchtungsmitteln, Geschmacksstoffen, Farbstoffen und Geruchsstoffen.

13. Zusammensetzung nach einem der Ansprüche 10 bis 12, ferner umfassend eine wirksame Menge eines Penetrationsförderers.

14. Pharmazeutische oder kosmetische Zusammensetzung nach einem der Ansprüche 10 bis 13, wobei der Wirkstoff ausgewählt ist für die Behandlung einer Erkrankung, deren Ätiologie bakteriell, fungal, viral, parasitär, entzündlich, autoimmun, allergisch, hormonell, bösartig und Kombinationen dieser ist; wobei der Wirkstoff ausgewählt ist für die Behandlung eines oberflächlichen Zustands; wobei der Wirkstoff ausgewählt ist für die Behandlung einer Erkrankung der Haut, der Schleimhaut, der Vagina oder des Rektums; wobei der Wirkstoff ausgewählt ist für die Behandlung einer Erkrankung, die ausgewählt ist aus der Gruppe bestehend aus Dermatose, Dermatitis, bakteriellen Infektionen, Pilzinfektionen, parasitären Infektionen, Virusinfektionen, Erkrankungen der Haarfollikel und Talgdrüsen, Akne, schuppenden papulären Erkrankungen, gutartigen Tumoren, bösartigen Tumoren, Reaktionen auf Sonnenlicht, bullöse Erkrankungen, Pigmentstörungen, Verhornungsstörungen, Druckgeschwüren, Schweißerzeugungsstörungen, entzündlichen Reaktionen, Xerose, Ichthyose, Allergien, Verbrennungen, Wunden, Schnittverletzungen und nicht dermatologischen Störungen, die auf die transdermale Verabreichung des Wirkstoffs reagieren; wobei der Wirkstoff ausgewählt ist für die Behandlung von Wunden, Verbrennungen, Schnittverletzungen und Geschwüren; wobei der Wirkstoff antibakteriell, antifungal oder antiviral ist; wobei der Wirkstoff ein Insektizid oder ein Insektenschutzmittel ist; wobei der Wirkstoff eine entzündungshemmende oder antiallergische Substanz ist; wobei der Wirkstoff ein Antikrebsmittel ist, ein photodynamisches Behandlungsmittel, ein Lokalanästhetikum, ein nichtsteroidaler Entzündungshemmer (NSAID); oder für die transdermale Verabreichung vorgesehen ist; wobei der Wirkstoff ein Retinoid, ein Antifaltenmittel oder ein Hautbleichmittel ist; wobei der Wirkstoff ausgewählt ist aus der Gruppe bestehend aus schwefelhaltigen Aminosäuren, Thiolverbindungen, Alpha-Hydroxysäuren, Milchsäure und deren Derivate und Salze, Glycolsäure und deren Derivate und Salze, Beta-Hydroxysäuren, Salizylsäure und Salizylsäuresalzen und Derivaten, Phytinsäure, Lipoinsäure, Lysophosphatidinsäure, Hautpeelingsubstanzen, Phenol, Resorcinol, Vitamin-B3-Verbindungen, Nikotinsäure und Nikotinsäuresalzen und -estern, Tocopherylnikotinat, Nikotinylaminsäuren, Nikotinylalkoholestern von Carboxylsäuren, Nikotininäure-N-Oxid und Niacinamid-N-Oxid, Retinoiden, Retinol, Retinal, Retinoinsäure, Retinylacetat, Retinylpalmitat, Retinylascorbat, Koffein, Theophilin, Pentoxyphillin, Dihydroxyacetonkojisäure, Arbutin, Nikotinsäure und deren Präkursor, Salze und Derivate, Arbutin, Ascorbinsäure und Salze und Derivate davon; wobei der Wirkstoff ein Pflanzenextrakt, ein Radikalfänger, ein Selbstbräunungsmittel, ein Anti-Aknewirkstoff, ein Figurbildner, eine das Haarwachstum beeinflussende Substanz, eine das Haarwachstum stimulierende Substanz, eine das Haarwachstum hemmende Substanz, ein Sonnenschutzmittel, ein anorganisches Sonnenschutzmittel, eine Kombination aus einem Hautbleichmittel und einem Sonnenschutzmittel oder eine Kombination aus einem Hautbleichmittel und einem anorganischen Sonnenschutzmittel ist.

15. Zusammensetzung nach einem der Ansprüche 10 bis 14, ferner umfassend ein Dekontaminierungsmittel, das ausgewählt ist aus der Gruppe bestehend aus einem Oxidationsmittel, lodin und Iodinverbindungen, Chlorhexidin, Bleichmitteln und Tensiden.

16. Zusammensetzung nach einem der Ansprüche 10 bis 15 zur Verwendung in einem Verfahren zur Behandlung des Körpers eines Menschen oder eines Tieres durch Therapie.

17. Verwendung einer Zusammensetzung nach einem der Ansprüche 10 bis 16 für die Herstellung eines Arzneimittels für die Behandlung einer Erkrankung, deren Ätiologie bakteriell, fungal, viral, parasitär, entzündlich, autoimmun, allergisch, hormonell, bösartig und Kombinationen dieser ist; wobei der Wirkstoff ausgewählt ist für die Behandlung eines oberflächlichen Zustands; wobei der Wirkstoff ausgewählt ist für die Behandlung einer Erkrankung der Haut, der Schleimhaut, der Vagina oder des Rektums; wobei der Wirkstoff ausgewählt ist für die Behandlung einer Erkrankung, die ausgewählt ist aus der Gruppe bestehend aus Dermatose, Dermatitis, bakteriellen Infektionen, Pilzinfektionen, parasitären Infektionen, Virusinfektionen, Erkrankungen der Haarfollikel und Talgdrüsen, Akne, schuppenden papulären Erkrankungen, gutartigen Tumoren, bösartigen Tumoren, Reaktionen auf Sonnenlicht, bullöse Erkrankungen, Pigmentstörungen, Verhornungsstörungen, Druckgeschwüren, Schweißerzeugungsstörungen, entzündlichen Reaktionen, Xerose, Ichthyose, Allergien, Verbrennungen, Wunden, Schnittverletzungen und nicht dermatologischen Störungen, die auf die transdermale Verabreichung des Wirkstoffs reagieren.

18. Verfahren zur kosmetischen Behandlung, umfassend das topische Verabreichen einer Zusammensetzung nach einem der vorstehenden Ansprüche, wobei die Zusammensetzung eine kosmetische Zusammensetzung ist, und wobei es sich bei der Behandlung um keine therapeutische Behandlung handelt.

## Revendications

1. Support pharmaceutique ou cosmétique moussant sans alcool, comprenant:
une composition moussante, comprenant :
environ 2 à 5 % en poids de la composition d'un solvant hydrophobe liquide non volatile et environ 80 à 98 % en poids de la composition d'eau ; ou environ 5 à 10 % en poids de la composition d'un solvant hydrophobe liquide non volatile et environ 75 à 95 % en poids de la composition d'eau ; ou environ 10 à 20 % en poids de la composition d'un solvant hydrophobe non volatile liquide et environ 60 à 90 % en poids de la composition d'eau ; ou environ 20 à 75 % en poids de la composition d'un solvant hydrophobe non volatile liquide et environ 25 à 75 % en poids de la composition d'eau ;
environ 0,1 à 5 % en poids de la composition d'un agent adjuvant de moussage choisi dans le groupe constitué des alcools gras, acides gras, alcools gras à substitution hydroxyle, acides gras à substitution hydroxyle, et acides gras et alcools gras comprenant au moins une double liaison dans leur chaîne d'atomes de carbone ;
environ 0,1 à 5 % en poids de la composition d'un agent tensioactif, dans lequel l'agent tensioactif est seulement non ionique, comprenant un ou plusieurs non ionique agents tensioactif, ou l'agent tensioactif est un mélange d'un ou plusieurs agents tensioactifs non ioniques et d'un ou plusieurs agents tensioactifs ioniques dans un rapport supérieur à 6:1, et
environ 0,1 à 5 % en poids de la composition d'un agent gélifiant d'eau ; et
un agent propulseur liquéfié, à une concentration d'environ 5 à environ 25 % en poids du support moussant, dans lequel l'agent propulseur liquéfié comprend un gaz hydrocarbure ou fluorocarbure;
dans lequel, la quantité combinée d'agent adjuvant de moussage, d'agent tensioactif et d'agent gélifiant d'eau est inférieure à environ 5 % en poids de la composition moussante;
dans lequel, lorsque la composition est libérée d'un récipient, elle fournit une mousse cassante à force de cisaillement appropriée pour une administration topique ou mucosale qui ne se brise pas facilement lors de décharge mais qui, par frottement sur la peau, s'effondre facilement.

2. Support moussant selon la revendication 1, dans lequel le solvant hydrophobe représente
environ de 5 à 10 % en poids de la composition,
environ de 10 à 20 % en poids de la composition, ou
environ de 20 à 75 % en poids de la composition.

3. Support moussant selon l'une quelconque des revendications précédentes, dans lequel le solvant hydrophobe comprend un mélange d'huile minérale et d'un émollient dans un rapport compris entre 2:8 et 8:2 sur une base en poids.

4. Support moussant selon l'une quelconque des revendications précédentes, dans lequel au moins 2 % de la composition moussant est une huile de silicone.

5. Support moussant selon l'une quelconque des revendications précédentes, dans lequel l'agent tensioactif ionique est un agent tensioactif anionique, un agent tensioactif cationique, un agent tensioactif amphotère ou zwitterionique.

6. Support moussant selon l'une quelconque des revendications précédentes, dans lequel l'agent tensioactif est un mélange d'un agent tensioactif non ionique et d'un agent tensioactif anionique dans un rapport de 20:1 à 1:1, ou l'agent tensioactif est un mélange d'un agent tensioactif non ionique et d'un agent tensioactif anionique dans un rapport de 100:1 à 6:1.

7. Support moussant selon l'une quelconque des revendications précédentes, dans lequel l'agent tensioactif a une valeur HLB supérieure à 9.

8. Support moussant selon l'une quelconque des revendications précédentes, dans lequel l'agent tensioactif comprend un ester de saccharose.

9. Support moussant selon l'une quelconque des revendications précédentes, dans lequel le solvant hydrophobe est choisi dans le groupe constitué des huiles végétales, huiles marines, huiles minérales, émollients, huiles de silicone, huiles thérapeutiques d'origine végétale et tout mélange de celles-ci en toute proportion.

10. Composition pharmaceutique ou cosmétique, comprenant :
un support moussant selon l'une quelconque des revendications précédentes ; et
un agent actif, dans laquelle lorsque la composition est une composition pharmaceutique, l'agent actif est présent dans une quantité thérapeutiquement efficace.

11. Composition pharmaceutique ou cosmétique selon la revendication 10, dans laquelle l'agent actif est un médicament ou un agent cosmétiquement efficace.

12. Composition pharmaceutique ou cosmétique selon la revendication 10 ou 11, comprenant en outre des excipients choisis dans le groupe constitué des antioxydants, humectants, aromatisants, colorants et agents odorants.

13. Composition selon l'une quelconque des revendications 10 à 12, comprenant en outre une concentration efficace d'un activateur de pénétration.

14. Composition pharmaceutique ou cosmétique selon l'une quelconque des revendications 10 à 13, dans laquelle l'agent actif est choisi pour le traitement d'une maladie, dont l'étiologie est bactérienne, fongique, virale, parasitaire, inflammatoire, auto-immune, allergique, hormonale, maligne et leurs combinaisons ; dans laquelle l'agent actif est choisi pour le traitement d'un état superficiel ; dans laquelle l'agent actif est choisi pour le traitement d'un problème de peau, de la membrane mucosale, du vagin ou du rectum ; dans laquelle l'agent actif est choisi pour le traitement d'un trouble choisi dans le groupe des dermatoses, dermatites, infections bactériennes, infections fongiques, infections parasitaires, infections virales, troubles des follicules pileux et des glandes sébacées, acné, rosacée, maladies papulaires exfoliatives, tumeurs bénignes, tumeurs malignes, réactions à la lumière du soleil, maladies bulleuses, troubles de la pigmentation, troubles de la kératinisation, escarres, troubles de la transpiration, réactions inflammatoires, xérose, ichtyose, allergie, brûlure, plaie, coupure, et troubles non dermatologiques, qui répondent à l'administration transdermique dudit agent actif ; dans laquelle l'agent actif est choisi pour le traitement des plaies, brûlures, coupures et ulcères ; dans laquelle l'agent actif est antibactérien, antifongique ou antiviral ; dans laquelle l'agent actif est un insecticide ou un insectifuge ; dans laquelle l'agent actif est un agent antiinflammatoire ou antiallergique ; dans laquelle l'agent actif est un agent anticancéreux, agent de thérapie photodynamique, anesthésique local, médicament anti-inflammatoire non stéroïdien (AINS) ; ou destiné à une administration transdermique ; dans laquelle l'agent actif est un rétinoïde, agent anti-rides, ou agent de blanchiment de la peau ; dans laquelle ledit agent actif est choisi dans le groupe comprenant les acides aminés contenant du soudre, composés thiols, acides alpha-hydroxy, acide lactique et ses dérivés et sels, acide glycolique et ses dérivés et sels, acides bêta-hydroxy, acide salicylique et ses sels et dérivés, acide phytique, acide lipoïque, acide lysophosphatidique, agents de décollement de la peau, phénol, résorcinol, composés de vitamine B3, niacinamide, acide nicotinique et ses sels et esters, nicotinate de tocophérol, acides aminés de nicotinyle, esters d'alcool nicotinylique d'acides carboxyliques, N-oxyde d'acide nicotinique et N-oxyde de niacinamide, rétinoïdes, rétinol, rétinal, acide rétinoïque, acétate de rétinyle, palmitate de rétinyle, ascorbate de rétinyle, caféine, théophiline, pentoxyphilline, acide kojique de dihydroxyacétone, arbutine, acide nicotinique et ses précurseurs, sels et dérivés, arbutine, acide ascorbique et ses sels et dérivés ; dans laquelle l'agent actif est un extrait de plantes, piégeur de radicaux, agent autobronzant, agent actif anti-acné, agent de formation de silhouette, agent influençant la croissance capillaire, agent stimulant la croissance capillaire, agent inhibiteur de la croissance capillaire, agent de protection solaire, agent de protection solaire inorganique, combinaison d'un agent de blanchiment de la peau et d'un agent de protection solaire, ou combinaison d'un agent de blanchiment de la peau et d'un agent de protection solaire inorganique.

15. Composition selon l'une quelconque des revendications 10 à 14, comprenant en outre un agent décontaminant choisi dans le groupe comprenant un agent oxydant, iode et composés d'iode, chlorhéxidine, agents de blanchiment et agents tensioactifs.

16. Composition selon l'une quelconque des revendications 10 à 15, destinée à être utilisée dans une méthode de traitement du corps humain ou animal par thérapie.

17. Utilisation d'une composition selon l'une quelconque des revendications 10 à 16, destinée à la fabrication d'un médicament pour le traitement d'une maladie, dont l'étiologie est bactérienne, fongique, virale, parasitaire, inflammatoire, auto-immune, allergique, hormonale, maligne et leurs combinaisons ; ou pour le traitement des dermatoses, dermatites, infections bactériennes, infections fongiques, infections parasitaires, infections virales, troubles des follicules pileux et des glandes sébacées, maladies papulaires exfoliatives, tumeurs bénignes, tumeurs malignes, réactions à la lumière du soleil, maladies bulleuses, troubles de la pigmentation, troubles de la kératinisation, escarres, troubles de la transpiration, réactions inflammatoires, xérose, ichtyose, allergie, brûlure, blessure, coupure, et troubles non dermatologiques, qui répondent à l'administration transdermique de l'agent actif.

18. Procédé de traitement cosmétique comprenant l'administration topique d'une composition selon l'une quelconque des revendications précédente, dans lequel la composition est une composition cosmétique et dans lequel le traitement est un traitement non thérapeutique.
